# EUROPÄISCHE PATENTSCHRIFT

(11) **EP 1 097 159 B1**
(45) Veröffentlichungstag und Bekanntmachung des Hinweises auf die Patenterteilung: **06.11.2002**
(21) Anmeldenummer: 99932841.2
(22) Anmeldetag: 07.07.1999
(51) Int. Cl.: C07F 9/38, A61K 31/66, C07F 9/40, C07F 9/572

(54) **PHOSPHIN- UND PHOSPHONSÄUREDERIVATE ALS ARZNEIMITTEL**
PHOSPHINOUS AND PHOSPHONIC ACID DERIVATIVES USED AS MEDICAMENTS
DERIVES D'ACIDE PHOSPHINIQUE ET D'ACIDE PHOSPHONIQUE UTILISES COMME MEDICAMENTS

(30) Priorität: 16.07.1998 DE 19831980; 12.05.1999 DE 19921680
(43) Veröffentlichungstag der Anmeldung: 09.05.2001
(73) Patentinhaber: Aventis Pharma Deutschland GmbH, 65929 Frankfurt am Main (DE)
(72) Erfinder: SCHUDOK, Manfred, D-65817 Eppstein (DE); SCHWAB, Wilfried, D-65207 Wiesbaden-Naurod (DE); ZOLLER, Gerhard, D-61137 Schöneck (DE); BARTNIK, Eckart, D-65205 Wiesbaden-Delkenheim (DE); BÜTTNER, Frank, D-67069 Ludwigshafen (DE); WEITHMANN, Klaus-Ulrich, D-65719 Hofheim (DE)
(86) Internationale Anmeldenummer: EP9904740
(87) Internationale Veröffentlichungsnummer: WO00004030

(56) Entgegenhaltungen:
- EP-A- 0 243 173
- EP-A- 0 757 037
- EP-A- 0 877 018
- EP-A- 0 877 019
- WO-A-97/44315

## Beschreibung

Die Erfindung betrifft neue Sulfonylaminophosphin- und phosphonsäurederivate, Verfahren zu ihrer Herstellung und Verwendung derselben als Arzneimittel.

In den Anmeldungen EP 0 606 046, WO 95/35276 und WO 96/27583 werden Arylsulfonaminohydroxamsäuren und deren Wirkung als Matrix-Metalloproteinase-Inhibitoren beschrieben. Spezielle Arylsulfonaminocarbonsäuren dienen als Zwischenprodukte zur Darstellung von Thrombin-Inhibitoren (EP 0 468 231) und Aldose-Reduktase-Inhibitoren (EP 0 305 947). In der Anmeldung EP 0 757 037 wird auch die Wirkung von Sulfonylaminocarbonsäure-Derivaten als Metalloproteinase-Inhibitoren beschrieben. Ferner hat sich die Arylsulfonylgruppe als eine effektive Schutzgruppe der Aminofunktion von α-Aminocarbonsäuren bewährt (R. Roemmele, H. Rapoport, J. Org. Chem. 53 (1988) 2367-2371).
In dem Bestreben, wirksame Verbindungen zur Behandlung von Bindegewebserkrankungen zu finden, wurde nun gefunden, daß die erfindungsgemäßen Sulfonylaminophosphin- und phosphonsäurederivate starke Inhibitoren von Metalloproteinasen sind. Dabei wird auf die Hemmung von Stromelysin (Matrix Metalloproteinase 3), der Neutrophilen Kollagenase (MMP-8) und der Aggrecanase besonderer Wert gelegt, da diese Enzyme beim Abbau der Proteoglykane, als wichtige Bestandteile des Knorpelgewebes, maßgeblich beteiligt sind (A. J. Fosang et al. J. Clin. Invest. 98 (1996) 2292-2299).
Der pathologische Verlust des Aggrecans, des Hauptproteoglykans des Knorpels, beinhaltet proteolytische Spaltungen in seiner interglobulären Domäne. Aminosäuresequenzanalysen von Proteoglykanmetaboliten, isoliert aus der Synovialflüssigkeit von Patienten, die an einer Gelenkschädigung, an Osteoarthrose oder an einer entzündlichen Gelenkerkrankung leiden, zeigten, daß eine proteolytische Spaltung bevorzugt zwischen den Aminosäuren Glu³⁷³ und Ala³⁷⁴ in der interglobulären Domäne des humanen Aggrecans stattfindet (Lohmander et al. Arthritis Rheum. 36, (1993), 1214-1222). Die proteolytische Aktivität, die für diese Spaltung verantwortlich ist, konnte bisher noch nicht identifiziert werden. Sie wird als "Aggrecanase" bezeichnet und kann zur Familie der Metalloproteinasen gerechnet werden.
Der erstmalige Expressionsnachweis der MT1-MMP im humanen Knorpelgewebe (Büttner et al. Arthritis Rheum. 40, 1997, 704-709), verbunden mit dem Nachweis, daß die katalytische Domäne dieses Enzyms an der "Aggrecanase"-Schnittstelle in dem rekombinanten Aggrecan-Fusionsprotein rAgg1ₘᵤₜ schneidet (Büttner et al. Biochem. J. 333, 1998, 159-165), führte zur Testung der hier beschriebenen starken Matrix Metalloproteinase-Inhibitoren bezüglich ihrer Wirkung gegenüber einer "Aggrecanase"-Aktivität. Dabei konnte mit verschiedenen Assay-Systemen gezeigt werden, daß die Sulfonylaminophosphin- und phosphönsäurederivate auch starke Inhibitoren für die "Aggrecanase"-Aktivität darstellen.

Die Erfindung betrifft daher die Verbindung der Formel I 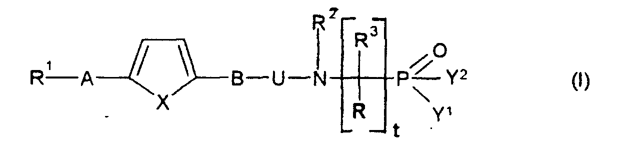 und/oder eine stereoisomere Form der Verbindung der Formel I und/oder ein physiologisch verträgliches Salz der Verbindung der Formel I, wobei
- R¹: für
1. Phenyl,
2. Phenyl, welches ein- oder zweifach substituiert ist durch
   2.1. (C₁-C₆)-Alkyl, gerade, cyclisch oder verzweigt,
   2.2. Hydroxy,
   2.3. (C₁-C₆)-Alkyl-C(O)-O-,
   2.4. (C₁-C₆)-Alkyl-O-,
   2.5. (C₁-C₆)-Alkyl-O-(C₁-C₄)-Alkyl-O-,
   2.6. Halogen,
   2.7. -CF₃,
   2.8. -CN,
   2.9. -NO₂,
   2.10. HO-C(O)-,
   2.11. (C₁-C₆)-Alkyl-O-C(O)-,
   2.12. Methylendioxo,
   2.13. R⁴-(R⁵ )N-C(O)-,
   2.14. R⁴-(R⁵)N- oder
   2.15 Heteroaromaten aus der Gruppe 3.1. bis 3.16,
3. einen Heteroaromaten aus der nachfolgenden Gruppe 3.1. bis 3.16, der unsubstituiert oder wie unter 2.1 bis 2.15 beschrieben substituiert ist,
   3.1. Pyrrol,
   3.2. Pyrazol,
   3.3. Imidazol,
   3.4. Triazol,
   3.5. Thiophen,
   3.6. Thiazol,
   3.7. Oxazol,
   3.8. Isoxazol,
   3.9. Pyridin,
   3.10. Pyrimidin,
   3.11. Pyrrolidin,
   3.12. Indol,
   3.13. Benzothiophen,
   3.14. Benzimidazol,
   3.15. Benzoxazol oder
   3.16. Benzothiazol, oder
4. -O-(C₁-C₆)-Alkyl, steht,
- R², R⁴ und R⁵: gleich oder verschieden sind und für
1. Wasserstoffatom,
2. (C₁-C₆)-Alkyl-,
3. HO-C(O)-(C₁-C₆)-Alkyl-,
4. Phenyl-(CH₂)ₙ-, worin Phenyl unsubstituiert oder ein- oder zweifach wie unter 2.1. bis 2.15. beschrieben substituiert oder durch -NH-C(O)-(C₁-C₃)-Alkyl substituiert ist und n die ganze Zahl Null, 1 oder 2 darstellt, oder
5. Picolyl stehen oder
6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist oder zwei benachbarte C-Atome des 4-bis 7-gliedrigen Rings Teil eines Benzylrestes sind,
- R und R³: gleich oder verschieden sind und für
1. Wasserstoffatom,
2. (C₁-C₁₀)-Alky(-, worin Alkyl unsubstituiert ist oder ein Wasserstöffatom des Alkylrestes durch -OH ersetzt ist,
3. (C₂-C₁₀)-Alkenyl-, worin Alkenyl gerade oder verzweigt ist,
4. R²-O-(C₁-C₆)-Alkyl-,
5. R²-S(O)ₙ-(C₁-C₆)-Alkyl-, wobei n die obengenannte Bedeutung hat,
6. R²-S(O)(=NH)-(C₁-C₆)-Alkyl-,
7. Rest der Formel IIo steht, 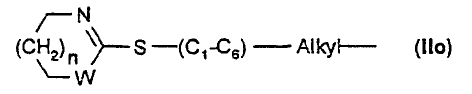 worin n die ganze Zahl Null, 1 oder 2 darstellt und W ein Stickstoff-, Sauerstoff-oder Schwefelatom darstellt,
8. Phenyl-(CH₂)ₘ-, worin m die ganze Zahl Null, 1, 2, 3, 4, 5 oder 6 ist und/oder ein Wasserstoffatom der -(CH₂)ₘ-Kette durch -OH ersetzt ist und Phenyl unsubstituiert oder ein- oder zweifach substituiert ist mit
   8.1 wie unter 2.1. bis 2.15. beschrieben,
   8.2 -O-(CH₂)ₘ-Phenyl, worin Phenyl unsubstituiert oder ein- oder zweifach wie unter 2.1. bis 2.15. beschrieben substituiert ist und m die ganze Zahl Null, 1, 2, 3, 4, 5 oder 6 darstellt,
   8.3 -C(O)-(CH₂)ₘ-Phenyl, worin Phenyl wie unter 8.2 definiert ist,
9. Heteroaryl-(CH₂)ₘ-, worin Heteroaryl wie unter 3.1. bis 3.16. definiert ist, m wie oben definiert ist und/oder ein Wasserstoffatom der -(CH₂)ₘ-Kette durch -OH ersetzt ist und Heteroaryl unsubstituiert oder ein- oder zweifach substituiert ist mit
   9.1 wie unter 2.1 bis 2.15 beschrieben,
   9.2 -CH(O),
   9.3 -SO₂-Phenyl, worin Phenyl unsubstituiert oder wie unter 8.2 definiert ist,
   9.4 -O-(CH₂)ₘ-Phenyl,
10. -(CH₂)ₘ-P(O)(OH)-(C₁-C₃)-Alkyl, worin m wie oben definiert ist,
11. charakteristischer Rest einer Aminosäure oder
12. R⁶-C(O)-(C₀-C₆)-Alkyl- steht, worin R⁶ für
   1. Wasserstoffatom,
   2. (C₁-C₆)-Alkyl-, worin Alkyl gerade, verzweigt oder cyclisch ist,
   3. Phenyl, worin Phenyl unsubstituiert oder wie unter 2.1 bis 2.15 beschrieben substituiert ist,
   4. Heteroaryl, worin Heteroaryl wie unter 3.1. bis 3.16. definiert und/oder wie unter 2.1 bis 2.15 beschrieben substituiert oder durch -(C₁-C₄)-Alkyl-COOH substituiert ist,
   5. -OH,
   6. -OR², worin R² die obengenannte Bedeutung hat,
   7. NR⁴-(R⁵) bedeutet, worin R⁴ und R⁵ wie oben definiert sind,
   8. Heteroaryl-(CH₂)ₘ-NH-, worin Heteroaryl wie unter 3.1 bis 3.16. definiert und/oder wie unter 2.1. bis 2.15 beschrieben substituiert ist und m wie oben definiert ist,
   9. R⁴-(R⁵)N-NH-, worin R⁴ und R⁵ wie oben definiert sind,
   10. HO-C(O)-CH(R³)-NH-, worin R³ wie oben definiert ist,
13. -(CH₂)ₚ-N(R⁹)(R¹⁰), worin p eine ganze Zahl Null, 1, 2, 3 oder 4 darstellt, worin R⁹ und R¹⁰ gleich oder verschieden sind und für
   1. Wasserstoffatom,
   2. Phenyl-(CH₂)ₘ-, worin Phenyl unsubstituiert oder ein- oder zweifach wie unter 2.1. bis 2.15. beschrieben substituiert ist und m die ganze Zahl Null, 1, 2 oder 3 ist,
   3. R^{x}-C(O)-, worin R^{x} für
      3.1 (C₁-C₆)-Alkyl-,
      3.2 (C₂-C₆)-Alkenyl-,
      3.3 Phenyl-(CH₂)ₘ-, worin Phenyl unsubstituiert oder ein- oder zweifach wie unter 2.1. bis 2.15. beschrieben substituiert ist und m die ganze Zahl Null, 1, 2 oder 3 darstellt, oder
      3.4 Heteroaryl-(CH₂)ₘ-, worin Heteroaryl wie unter 3.1.bis 3.16. definiert und/oder wie unter 2.1 bis 2.15 beschrieben substituiert ist und m die ganze Zahl Null, 1, 2 oder 3 darstellt, steht,
   4. R^{x}-O-C(O)-, worin R^{x} wie oben genannt definiert ist,
   5. R^{x}-CH(NH₂)-C(O)-, worin R^{x} wie oben genannt definiert ist,
   6. R⁸-N(R⁷)-C(O)-, worin R⁸ für
      6.1 Wasserstoffatom
      6.2 (C₁-C₆)-Alkyt-,
      6.3 Phenyl-(CH₂)ₘ, worin Phenyl unsubstituiert oder ein- oder zweifach wie unter 2.1. bis 2.15. beschrieben substituiert ist und m die ganze Zahl Null, 1,2 oder 3 darstellt, oder
      6.4 Heteroaryl-(CH₂)ₘ, worin Heteroaryl wie unter 3.1. bis 3.16. definiert und/oder wie unter 2.1 bis 2.15 beschrieben substituiert ist und m die ganze Zahl Null, 1, 2 oder 3 darstellt, stehtund worin R⁷ Wasserstoffatom oder (C₁-C₆)-Alkyl- bedeutet oder worin R⁷ und R⁸ zusammen mit dem Stickstoffatom an das sie gebunden sind einen 4- bis 7-gliedrigen Ring bilden und der Ring unsubstituiert ist oder ein Kohlenstoffatom im Ring durch -O-, -S- oder -NH- ersetzt ist,
   7. R^{x}-SO₂-, worin R^{x} wie oben genannt definiert ist,
   8. R^{x}-NH-C(=NR⁷)-, worin R^{x} und R⁷ wie oben genannt definiert sind oder für
      8.1 (C₁-C₆)-Alkyl-C(O)-,
      8.2 -NO₂ oder
      8.3 -SO₂-(CH₂)_{q}-Phenyl, worin Phenyl unsübstituiert oder ein- oder zweifach wie unter 2.1. bis 2.15. beschrieben substituiert ist und q die ganze Zahl Null, 1,2 oder 3 ist, stehen,
   9. -SO₂-(CH₂)_{q}-Phenyl-Phenyl, worin Phenyl unsubstituiert oder ein-oder zweifach wie unter 2.1. bis 2.15. beschrieben substituiert ist und q die ganze Zahl Null, 1,2 oder 3 ist, darstellen, oder
   10. den Rest der Formel IIp steht, 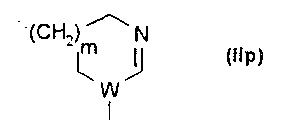 worin m die ganze Zahl Null, 1, 2 oder 3 bedeutet und W für ein Stickstoffatom steht, oder
   R⁹ und R¹⁰ zusammen mit dem Stickstoffatom an das sie gebunden sind einen Ring der Teilformel IIa bis IIn bilden, 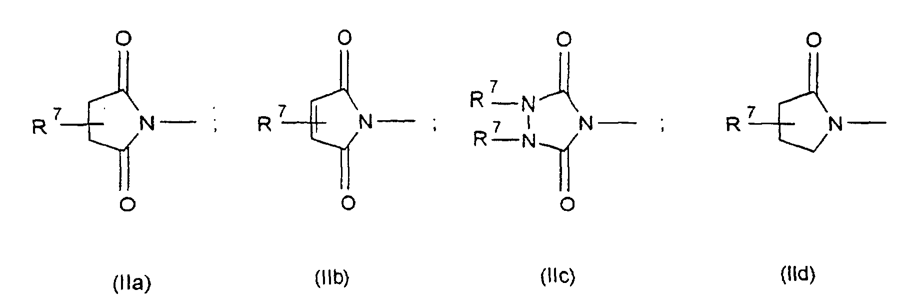 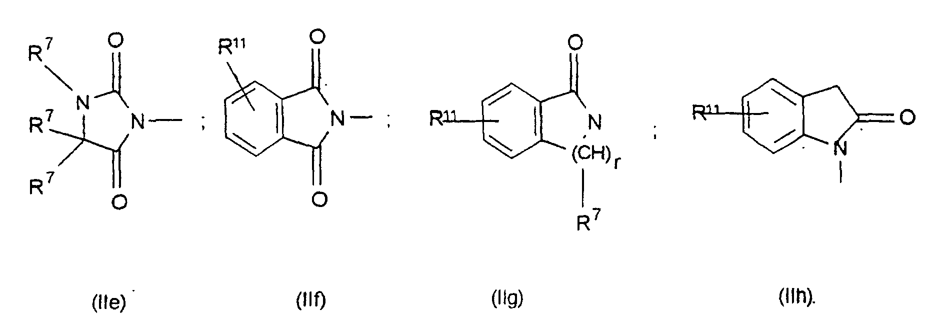 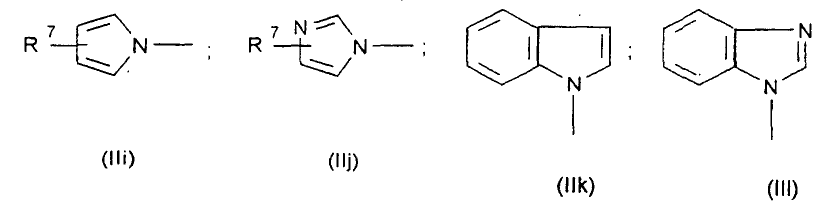 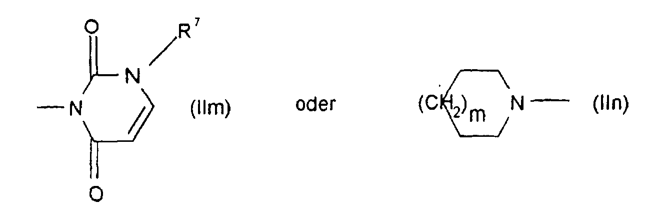 wobei r die ganze Zahl 1 oder 2 bedeutet, R¹¹ einen Rest wie unter 2.1. bis 2.15 beschrieben bedeutet, und R⁷ und m die oben genannte Bedeutung haben,
14. -OH,
15. =O oder
16. (C₁-C₆)-Alkyl- steht, oder
   ein -C(R)(R³)- Rest für, -NH- oder -NR²- steht, worin R² wie oben definiert ist, und
   t für eine ganze Zahl 1, 2, 3 oder 4 steht, oder
R² und R³ bilden zusammen einen Ring mit exocyclischem Phosphin- oder Phosphonsäurerest der Teilformel II, 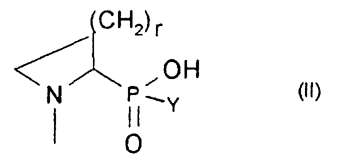 worin r die ganze Zahl Null, 1, 2 oder 3 darstellt und/oder eines der Kohlenstoffatome im Ring durch -O-, -S- oder -(R⁷)N- ersetzt ist, worin
R⁷ für
1. Wasserstoffatom,
2. (C₁-C₆)-Alkyl,
3. Phenyl, worin Phenyl unsubstituiert oder wie unter 2.1 bis 2.15 beschrieben substituiert ist,
4. Benzyl, worin Benzyl unsubstituiert oder wie unter 2.1 bis 2.15 beschrieben substituiert ist, oder
5. R²N-C(=NH)- steht, wobei R² die obengenannte Bedeutung hat, und/oder die Kohlenstoffatome im Ring der Teilformel II ein- oder mehrfach durch (C₁-C₆)-Alkyl-, Phenyl-, Phenyl-(CH₂)ₘ- oder HO- substituiert sind,
- U: für -SO₂- oder -CO- steht,
- Y¹ und Y²: gleich oder verschieden sind und unabhängig voneinander für
a) Wasserstoffatom,
b) -OH,
c) -(C₁-C₄)-Alkyl, worin Alkyl gerade oder verzweigt ist,
d) -(CH₂)ᵤ-Phenyl, worin u Null oder 1 ist,
e) -O-(C₁-C₄)-Alkyl, worin Alkyl gerade oder verzweigt ist, oder
f) -O-(CH₂)ₛ-Phenyl, worin s Null oder 1 ist, steht,
- A: für
a) eine kovalente Bindung,
b) -O-,
c) -CH=CH- oder
d) -C≡C- steht,
- B: für
a) -(CH₂)ₒ-, worin o die ganze Zahl Null, 1, 2, 3 oder 4 bedeutet,
b) -O-(CH₂)ₚ, worin p eine ganze Zahl von 1 bis 5 bedeutet, oder
c) -CH=CH- steht, und
- X: für -CH=CH-, Sauerstoffatom oder Schwefelatom steht.

Bevorzugt ist eine Verbindung der Formel I, wobei
- R¹: für
1. Phenyl oder
2. Phenyl, welches einfach substituiert ist durch
   2.1. (C₁-C₆)-Alkyl-, worin Alkyl gerade, cyclisch oder verzweigt ist,
   2.2. -OH,
   2.3. -C(O)-OH,
   2.4. -O-(C₁-C₆)-Alkyl,
   2.5. Pyrrolidin,
   2.6. Halogen oder
   2.7. -CF₃ steht, oder
3. -O-(C₁-C₆)-Alkyl, steht
R², R⁴ und R⁵ gleich oder verschieden sind und für Wasserstoffatom oder (C₁-C₆)-Alkylstehen,
- R: für Wasserstoffatom steht,
- R³: für
1. (C₁-C₆)-Alkyl-, worin Alkyl gerade, verzweigt oder cyclisch ist, und/oder worin ein Wasserstoffatom des Alkylrestes durch -OH ersetzt ist,
2. R²-S(O)ₙ-(C₁-C₆)-Alkyl-, worin R²(C₁-C₆)-Alkyl- oder Phenyl-(CH₂)ₙ-bedeutet und n die ganze Zahl Null oder 1 ist,
3. -(CH₂)ₘ-Phenyl, worin Phenyl unsubstituiert oder ein- oder zweifach wie unter 2.1. bis 2.15. beschrieben substituiert ist und/oder ein Wasserstoffatom der (CH₂)ₘ- Kette durch -OH ersetzt ist und m die ganze Zahl 1, 2, 3, 4 oder 5 darstellt,
4. -(CH₂)ₘ-Heteroaryl, worin Heteroaryl die unter 3.3, 3.5, 3.6, 3.9 oder 3.11 genannte Bedeutung hat und/oder wie unter 2.1. bis 2.15. beschrieben substituiert ist und/oder ein Wasserstoffatom der -(CH₂)ₘ-Kette durch -OH ersetzt ist und m die ganze Zahl 1, 2, 3 oder 4 ist,
5. charakteristischer Rest einer Aminosäure oder
6. -(CH₂)ₚ-N(R⁹)(R¹⁰), worin p eine ganze Zahl Null, 1 oder 2 darstellt, worin R⁹ und R¹⁰ gleich oder verschieden sind und für Wasserstoffatom oder -SO₂-(CH₂)_{q}-Phenyl-Phenyl, worin Phenyl unsubstituiert oder ein-oder zweifach wie unter 2.1. bis 2.15. beschrieben substituiert ist und q die ganze Zahl Null, 1,2 oder 3 ist, darstellen, steht oder
7. R⁶-C(O)- steht, worin R⁶ für
   7.1. -OH,
   7.2. R²O-, worin R² wie oben definiert ist, oder
   7.3. R⁴-(R⁵)N- steht, worin R⁴ und R⁵ wie oben definiert sind,
8. Wasserstoffatom,
9. -OH,
10. =O oder
11. (C₁-C₆)-Alkyl- steht, oder
   ein -C(R)(R³)- Rest für, -NH- oder -NR²- steht, worin R² wie oben definiert ist, und
   t für eine ganze Zahl 1, 2, 3 oder 4 steht,
- U: für -SO₂- steht,
- Y¹: für -OH steht,
- Y²: für
a) -O-(C₁-C₄)-Alkyl, worin Alkyl gerade oder verzweigt ist,
b) -OH oder
c) -(C₁-C₄)-Alkyl, worin Alkyl gerade oder verzweigt ist, steht,
- A: für eine kovalente Bindung oder -O- steht,
- B: für eine kovalente Bindung oder -(C₁-C₄)-Alkyl steht und
- X: für -CH=CH- steht.

Besonders bevorzugt ist eine Verbindung der Formel I, wobei
- R¹: für 1. Phenyl steht, welches einfach durch Halogen substituiert ist,
- R²: für Wasserstoffatom steht,
- R: für Wasserstoffatom steht,

- R³: für
1. (C₁-C₄)-Alkyl-,
2. -Phenyl, worin Phenyl unsubstituiert oder ein- oder zweifach durch -CF₃ oder -COOH substituiert ist,
3. Wasserstoffatom,
4. -OH oder
5. -NH-SO₂-Phenyl-Phenyl, worin Phenyl unsubstituiert oder durch Halogen substituiert ist, steht,

t für eine ganze Zahl 1, 2, 3 oder 4 steht,
- U: für -SO₂-steht,
Y¹ und Y² für -OH oder -O-CH₃ steht,
A für eine kovalente Bindung steht,
B für eine kovalente Bindung oder -(CH₂)ₒ- steht, worin o 1, 2 oder 3 bedeutet und
X für -CH=CH- steht.

Insbesondere bevorzugt sind die Verbindungen ( R )- [1-(4'-Chlorbiphenyl-4-sulfonylamino)-2-methylpropyl]-phosphonsäure, [3-(4'-Chlorbiphenyl-4-sulfonylamino)-1-hydroxy-3-(4-trifluormethyl-phenyl)-propyl]-phosphonsäuredimethylester oder [1-(4'-Chlor-biphenyl-4-sulfonylamino)-3-methyl-butyl]-phosphonsäure.

Mit dem Begriff "R⁴ und R⁵ zusammen mit der ringständigen Aminogruppe einen 4- bis 7-gliedrigen Ring bilden und/oder eines der Kohlenstoffatome durch -O-, -S- oder -NHersetzt ist" werden Reste verstanden, die sich beispielsweise von Azetidin, Pyrrol, Pyrrolin, Pyridin, Azepin, Piperidin, Oxazol, Isoxazol, Imidazol, Indolin, Pyrazol, Thiazol, Isothiazol, Diazepin, Thiomorpholin, Pyrimidin oder Pyrazin ableiten. Unter dem Begriff "Halogen" wird Fluor, Chlor, Brom oder Jod verstanden. Unter dem Begriff "Alkyl" oder "Alkenyl" werden Kohlenwasserstoffreste verstanden, deren Kohlenstoffketten geradkettig oder verzweigt sind. Cyclische Alkylreste sind beispielsweise 3- bis 6-gliedrige Monocyclen wie Cyclopropyl, Cyclobutyl, Cyclopentyl oder Cyclohexyl. Ferner können die Alkenylreste auch mehrere Doppelbindungen enthalten.
Die allgemeine Strukturformel von α-Aminosäuren ist wie folgt:

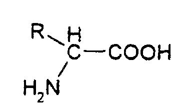

Die α-Aminosäuren unterscheiden sich untereinander durch den Rest R, der im Rahmen der vorliegenden Anmeldung als "charakteristischer Rest " einer Aminosäure bezeichnet wird.

Die Ausgangsstoffe der chemischen Umsetzungen sind bekannt oder lassen sich nach literaturbekannten Methoden leicht herstellen. Die als Ausgangsstoffe für die Synthese der erfindungsgemäßen Verbindungen verwendeten Aminophosphin- und -phosphonsäuren sind, wenn nicht im Einzelfall käuflich erhältlich, nach bekannten Methoden synthetisierbar (R. S. Rogers, M. K. Stern, Synlett **1992**, 708; P. P. Giannousis, P. A. Bartlett, J. Med. Chem. **30**, 1603 (1987); J. P. Genet, M. Uziel, A. M. Touzin, S. Roland, S. Thorimbert, S. Tanier, Tetrahedron Lett. **33**, 77 (1992); E. K. Baylis, C. D. Campbell, J. G. Dingwall, J. Chem. Soc. Perkin Trans. 1, **1984**, 2845).

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) eine Aminophosphin- oder phosphonsäure der Formel III, 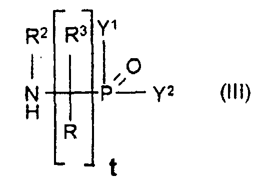 worin R², Y¹, Y², R und R³ wie in Formel I definiert sind, mit einem Sulfonsäureoder Carbonylderivat der Formel IV, 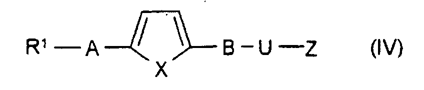 worin R¹, A, X, U und B wie in Formel I definiert sind und Z ein Halogenatom, Imidazolyl oder -OR⁸ bedeutet, worin R⁸ Wasserstoffatom, (C₁-C₆)-Alkyl, Phenyl oder Benzyl, gegebenenfalls substituiert darstellt, in Gegenwart einer Base oder gegebenenfalls eines wasserentziehenden Mittels zu einer Verbindung der Formel I umsetzt, oder
b) einen Aminophosphin- oder phosphonsäureester der Formel V, 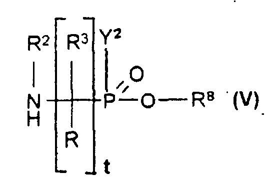 worin R², R³, t, Y² und R⁸ die obengenannte Bedeutung haben,
   mit einem Sulfonsäure- oder Carbonylderivat der Formel IV zu einer Verbindung der Formel VI 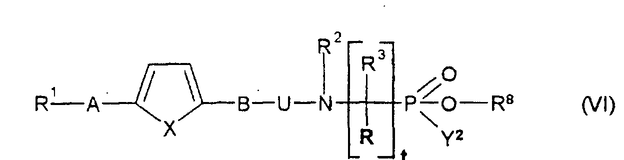 umsetzt, und die Verbindung der Formel VI unter Abspaltung des Restes R⁸, bevorzugt in Gegenwart einer Base oder Säure in eine Verbindung der Formel I umwandelt, oder
c) die Verbindung der Formel VII, 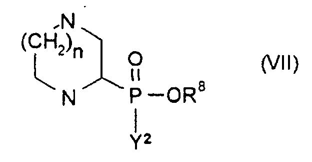 wobei n die ganze Zahl Null, 1 oder 2 darstellt,
   mit Hilfe einer Schutzgruppe E zu einer Verbindung der Formel VIII umsetzt, 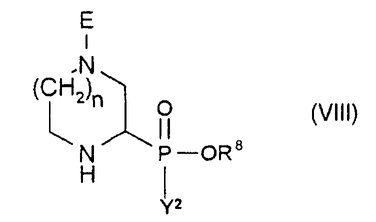 und die Verbindung der Formel VIII mit einer Verbindung der Formel IV in eine Verbindung der Formel IX überführt, 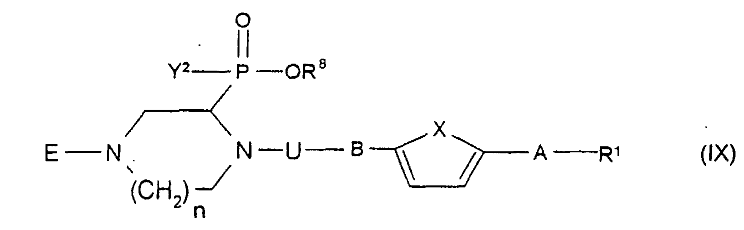 und anschließend die Verbindung der Formel IX unter Abspaltung der Schutzgruppe E und des Restes R⁸ mit Hilfe geeigneter Spaltreagenzien in die Verbindung der Formel I überführt, oder
d) eine nach den Verfahren a), b) oder c) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
e) die nach den Verfahren a), b), c) oder d) hergestellte Verbindung der Formel I entweder in freier Form isoliert oder im Falle des Vorliegens von sauren oder basischen Gruppen in physiologisch verträgliche Salze umwandelt.

Als geeignete Schutzgruppe E werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Aloc) oder vom Säureamid-Typ insbesondere Formyl, Acetyl oder Trifluoracetyl sowie vom Alkyl-Typ beispielsweise Benzyl.

Als Verbindungen der Formel III, bei denen R² ein Wasserstoffatom und R³ der charakteristische Rest einer Aminosäure bedeutet, werden vorzugsweise die charakteristischen Reste der folgenden natürlich vorkommenden α-Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure eingesetzt. Als Verbindungen der Formel III, bei denen R² ein Wasserstoffatom und R³ der charakteristische Rest einer Aminosäure bedeutet, werden vorzugsweise die charakteristischen Reste beispielsweise der folgenden nicht natürlich vorkommenden Aminosäuren 2-Aminoadipinsäure, 2-Aminobuttersäure, 2,4-Diaminobuttersäure, 2-Aminoisobuttersäure, 2,3-Diaminopropionsäure, 1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Aminoheptansäure, Pipecolinsäure, Hydroxylysin, Sarkosin,. N-Methylisoleucin, 6-N-Methyllysin, N-Methylvalin, Norvalin, Norleucin, Ornithin, allo-Isoleucin, allo-Threonin, 4-Hydroxyprolin, 3-Hydroxyprolin, allo-Hydroxylysin, 3-(2-Naphtyl)-alanin, 3-(1-Naphtylalanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, Citrullin, Phosphinothricin, 4-Fluorphenylalanin, 3-Fluorphenylalanin, 2-Fluorphenylalanin, 4-Chlorphenylalanin, 4-Nitrophenylalanin, 4-Aminophenylalanin, Cyclohexylalanin, 5-Fluortryptophan, 5-Methoxytryptophan, Methionin-Sulfon, Methionin-Sulfoxid oder NH₂-NH-CONH₂ ggf. substituiert, eingesetzt. Bei natürlichen aber auch nicht natürlichen vorkommenden Aminosäuren, die eine funktionelle Gruppe wie Amino, Hydroxy, Carboxy, Mercapto, Guanidyl, Imidazolyl oder Indolyl in der Seitenkette R³ haben, kann diese Gruppe auch geschützt sein.

Für den Fall eines Imidazols-Restes in R³ dient beispielsweise das für die Sulfonamidbildung eingesetzte Sulfonsäurederivat der Formel IV als Schutzgruppe des Imidazol-Stickstoffs, die sich insbesondere in Gegenwart von Basen wie Natronlauge wieder abspalten läßt.
Zur Darstellung von Verbindungen der Formel I,in denen R² und R³ zusammen einen Ring der Teilstruktur II bilden, werden als Ausgangsstoffe der Formel III beispielsweise 2-Methylpropyl-phosphonsäure, Piperidin-2-phosphonsäure, Piperazin-2-phosphonsäureoder Hexahydropyridazin-3-phosphonsäure benutzt, wobei insbesondere der Stickstoff in 4-Stellung der Piperazin-2-phosphonsäure durch eine Schutzgruppe Z, beispielsweise Benzyloxycarbonyl- oder tert.-Butyloxycarbonyl wie in der Verfahrensvariante c) beschrieben oder durch einen Rest R⁷ substituiert sein kann.

Als Ausgangsprodukte zur Darstellung der Sulfonsäurederivate der Formel IV dienen bevorzugt Sulfonsäuren oder deren Salze der Formel X, beispielsweise 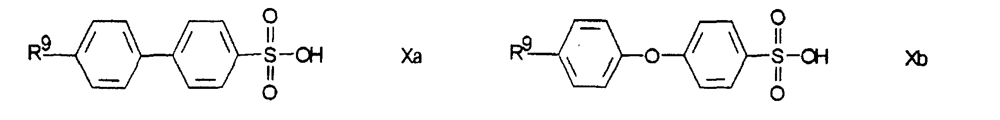 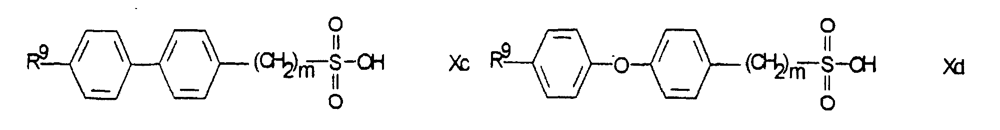 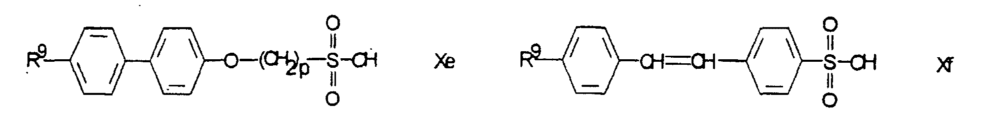 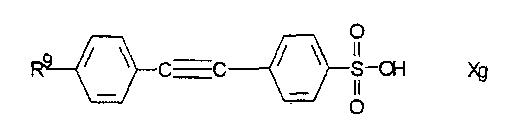 wobei R⁹ ein unter 2.1. bis 2.15. beschriebener Rest bedeutet.

Zur Herstellung der Arylsulfonsäuren der Formeln Xa und b bedient man sich vorzugsweise der im Houben-Weyl "Methoden der Organischen Chemie" Band 9, S. 450-546 beschriebenen Sulfonierungsverfahren mit konzentrierter Schwefelsäure gegebenenfalls in Gegenwart eines Katalysators, Schwefeltrioxids und seinen Additionsverbindungen oder Halogensulfonsäuren, wie Chlorsulfonsäure. Besonders im Falle der Diphenylether der Formel Xb hat sich die Verwendung von konzentrierter Schwefelsäure und Essigsäureanhydrid als Lösemittel (vergl. C.M. Suter, J. Am. Chem. Soc. 53 (1931) 1114), oder die Umsetzung mit überschüssiger Chlorsulfonsäure (J.P. Bassin, R. Cremlyn und F. Swinbourne; Phosphorus, Sulfur and Silicon 72 (1992) 157) bewährt. Sulfonsäuren gemäß der Formel Xc, Xd oder Xe lassen sich in an sich bekannter Weise herstellen, in dem man das entsprechende Arylalkylhalogenid mit Sulfiten wie Natriumsulfit oder Ammoniumsulfit in wäßriger oder wäßrig/alkoholischer Lösung umsetzt, wobei die Umsetzung in Gegenwart von Tetraorganoammoniumsalzen wie Tetrabutylammoniumchlorid beschleunigt werden kann.

Als Sulfonsäurederivate gemäß Formel IV finden insbesondere die Sulfonsäurechloride Verwendung. Zu ihrer Herstellung werden die entsprechenden Sulfonsäuren, auch in Form ihrer Salze wie Natrium-, Ammonium- oder Pyridiniumsalze in bekannter Weise mit Phosphorpentachlorid oder Thionylchlorid ohne oder in Gegenwart eines Lösemittels wie Phosphoroxytrichlorid oder eines inerten Lösemittels wie Methylenchlorid Cyclohexan oder Chloroform im allgemeinen bei Reaktionstemperaturen von 20°C bis zum Siedepunkt des verwendeten Reaktionsmediums umgesetzt.

Die Umsetzung der Sulfonsäurederivate der Formel IV mit den Aminosphosphonsäuren der Formeln III, V oder VII gemäß Verfahrensvarianten a), b) oder c) verläuft vorteilhaft nach Art der Schotten-Baumann-Reaktion. Als Base eignen sich dafür besonders Alkalihydroxide wie Natriumhydroxid, aber auch Alkaliacetate, -hydrogencarbonate,-carbonate und Amine. Die Umsetzung findet in Wasser und/oder in einem mit Wasser mischbaren oder nicht mischbaren Lösemittel wie Tetrahydrofuran (THF), Aceton, Dioxan oder Acetonitril statt, wobei die Reaktionstemperatur im allgemeinen von -10°C bis 50°C gehalten wird. Für den Fall, daß die Reaktion im wasserfreien Medium durchgeführt wird, findet vor allem Tetrahydrofuran oder Methylenchlorid, Acetonitril oder Dioxan in Gegenwart einer Base, wie Triethylamin, N-Methylmorpholin, N-Ethyl- oder Diisopropylethylamin Verwendung, eventuell in Gegenwart von N,N-Dimethylaminopyridin als Katalysator.

In einer anderen Variante kann man die Aminocarbonsäuren der Formel III, IV oder VII zuerst mit Hilfe eines Silylierungsmittels wie Bis-trimethylsilyltrifluoracetamid (BSTFA) in ihre silylierte Form überführen und sie dann mit Sulfonsäurederivaten zu Verbindungen der Formel I umsetzen.

Die Herstellung physiologisch verträglicher Salze aus zur Salzbildung befähigten Verbindungen der Formel I, einschließlich deren stereoisomeren Formen, erfolgt in an sich bekannter Weise. Die Phosphon- oder Phosphinsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls. stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Enzymen wie Metalloproteinasen oder Aggrecanase, beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.
Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 400-MHz-Gerät der Firma Bruker oder einem 200 MHz-Gerät der Firma Varian aufgenommen worden, in der Regel mit Tetramethylsilan (TMS) als internem Standard und bei Raumtemperatur (RT). Die verwendeten Lösemittel sind jeweils angegeben. Endprodukte werden in der Regel durch massenspektroskopische Methoden (FAB-, ESI-MS) bestimmt, angegeben sind jeweils der Hauptpeak. Temperaturangaben in Grad Celsius, RT bedeutet Raumtemperatur (22°C bis 26°C). Verwendete Abkürzungen sind entweder erläutert oder entsprechen den üblichen Konventionen.

### Beispiel 1 ( R )- [1-(4'-Chlorbiphenyl-4-sulfonylamino)-2-methylpropyl]-phosphonsäure

250 mg (1,6 mmol) von (R)-(1-Amino-2-methylpropyl)-phosphonsäure wurden in 6 ml einer 1 M NaOH und 6 ml Tetrahydrofuran gelöst. Anschließend gab man 560 mg (1,96 mmol) von 4-Chlorbiphenyl-4'-sulfonylchlorid hinzu und rührte bei 22 °C über Nacht. Das Reaktionsgemisch wurde eingeengt, mit 2 M HCI angesäuert und mit Essigsäureethylester extrahiert. Die als Nebenprodukt entstehende 4-Chlorbiphenyl-4'sulfonsäure fiel aus und wurde abgetrennt. Nach dem Trocknen und Einengen der Essigester-Phase erhielt man einen Feststoff.
Ausbeute: 136 mg (21%); Molmasse: 403,83
¹H-NMR: in DMSO-d6; 10,8 (s,br, 2 H); 7,91; 7,82; 7,76; 7,63 7,56 (5 d, 9 H); 3,06 (m, 1H); 1,98 (m, 1H); 0,87; 0,80 (dd, 6H); MS (ESI; M + Na⁺): 425,9

### Beispiel 2 ( R,S )- [1-(4'-Chlorbiphenyl-4-sulfonylamino)-1-phenylmethyl]-phosphonsäure-monoethylester

830 mg (3,85 mmol) des (R,S)-( Aminophenylmethyl)-phosphonsäuremonoethylesters wurden in 6 ml einer 2 M NaOH und 10 ml Tetrahydrofuran gelöst. Anschließend gab man 1,44 g (5,01 mmol) von 4-Chlorbiphenyl-4'-sulfonylchlorid hinzu und rührte bei 22 °C über Nacht. Der entstandene Niederschlag wurde abgetrennt und in heißem Wasser/Essigsäureethylester verteilt. Nach dem Ansäuern mit HCI auf pH 1 bis 2 wurde die Essigsäureethylester-Phase abgetrennt und eingeengt. Es verbleibt ein Feststoff.
Ausbeute: 610 mg (34 %); Molmasse: 465
¹H-NMR: in DMSO-d6; 8,66 (s, br, 1 H); 7,57 (m, 9 H); 7,16 (m, 2 H); 7,01 (m, 3 H); 4,58 (dd, 1 H) 3,85 (m, 2H); 1,11 (m, 3H); MS (FAB; M⁺, M + Na⁺): 466,0; 488,0

### Beispiel 3 (R,S)-[(4'-Chlorbiphenyl-4-sulfonylamino)-phenylmethyl]-phosphonsäure

320 mg (0,69 mmol) des Monoethylesters aus Beispiel 2 wurden in 6 ml Dichlormethan gelöst und bei 0 °C mit 0,36 ml (2,75 mmol) Trimethylsilylbromid versetzt. Nach 4 h bei RT wurde die Reaktionsmischung am Rotationsverdampfer zur Trockne eingeengt und der verbleibende Rückstand in Wasser aufgenommen. Feststoffe wurden abgetrennt und die wässrige Phase wurde gefriergetrocknet.
Ausbeute: 257 mg (80 %); Molmasse: 436,8 g/mol
¹H-NMR: DMSO-d₆; 7,6 (m, 8 H); 7,2 (m, 2 H); 7,0 (m, 3 H); 4,2 (m, 1 H)
MS (ESl⁻): 436,0

### Beispiel 4 (R,S)-[1-(4'-Chlorbiphenyl-4-sulfonylamino)-2-(1H-indol-3-yl)-ethyl]-phosphonsäure

150 mg (0,274mmol) des entsprechenden Diethylesters wurden in 4 ml Dichlormethan gelöst und bei Raumtemperatur mit 0,11 ml (0,82 mmol) Trimethylsilylbromid versetzt. Nach 3 h wurde die Reaktionsmischung am Rotationsverdampfer zur Trockne eingeengt, der verbleibende Rückstand mit Diisopropylether behandelt und der Feststoff durch Filtration abgetrennt.
Ausbeute: 42 mg (33 %); Molmasse: 490,92
¹H-NMR: DMSO-d₆; 10,4 (s, 2 H); 7,9; 7,68; 7,55 (3 d, 5 H); 7,3; 6,9 (2 m, 8 H); 3,7 (m, 1 H); 3,2-2,6 (2 m, 4H); MS (ESl⁺): 491,0

### Beispiel 5 (R,S)-[1-(4'-Chloro-biphenyl-4-sulfonylamino)-ethyl]-phosphonsäure

Zu 178 mg (1,4 mmol) (R.S)-1-Amino-ethyl-phosphonsäure in 30 ml Acetonitril wurden unter Stickstoff 733 mg (2.8 mmol) N,O-Bis-trimethylsilyl-trifluoracetamid zugegeben und 2 h unter Rückfluß erhitzt. Nach Abkühlen auf 15°C wurden 490 mg (1,7 mmol) 4'-Chlorbiphenyl-4-sulfonylchlorid in 15 ml Acetonitril zugegeben. Man rührte 3 h bei RT, engte ein, versetzte mit Methanol und enget erneut ein. Der Rückstand wurde über Kieselgel mit Methylenchlorid/Methanol 75:25 und 1% Essigsäure chromatographiert.
Ausbeute: 60 mg (11%), Molmasse: 375.77
¹H-NMR: DMSO-d₆; 1.0-1.2 (m, 3H), 3.35-3.55 (m, 1H), 7.5 (d, 2H), 7.68 (d, 2H), 7.8 (d, 2H), 8.0 (d, 2H); MS (ESl⁻): 374.1

### Beispiel 6 (R,S)- [1-(4'-Chloro-biphenyl-4-sulfonylamino)-3-methyl-butyl]-phosphonsäure

Zu 222 mg (1 mmol) (R,S)-1-Amino-3-methylbutyl-phosphonsäure hydrochlorid in 30 ml Acetonitril wurden unter Stickstoff 516 mg (2 mmol) N,O-Bis-trimethylsilyl-trifluoracetamid zugegeben und 2 h unter Rückfluß erhitzt. Nach Abkühlen auf 15°C wurden 345 mg (1,2 mmol) 4'-Chlorbiphenyl-4-sulfonylchlorid in 15 ml Acetonitril zugegeben. Man rührt 3,5 h bei RT, engte ein, versetzte mit Methanol und engte ein. Der Rückstand wurde über RP18 mit Acetonitril/Wasser (enthält 0,1% Trifluoressigsäure), Gradient 10% bis 100% Acetonitril chromatographiert.
Ausbeute: 75 mg (18%), Molmasse: 417,85; MS (ESl⁻): 416,1

Die in der nachfolgenden Tabelle 1 definierten Verbindungen wurden analog zu den Beispielen 1 bis 6 hergestellt.

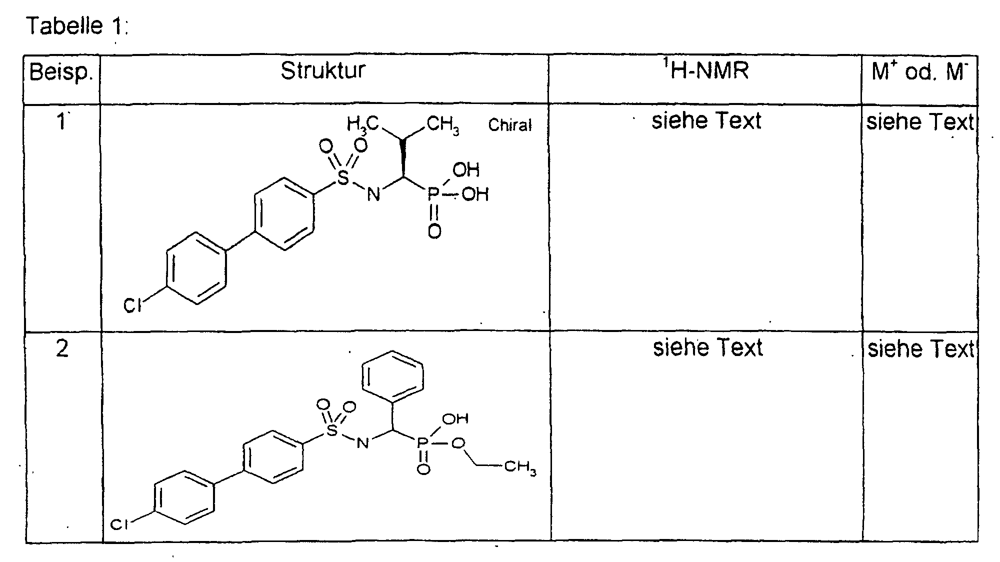

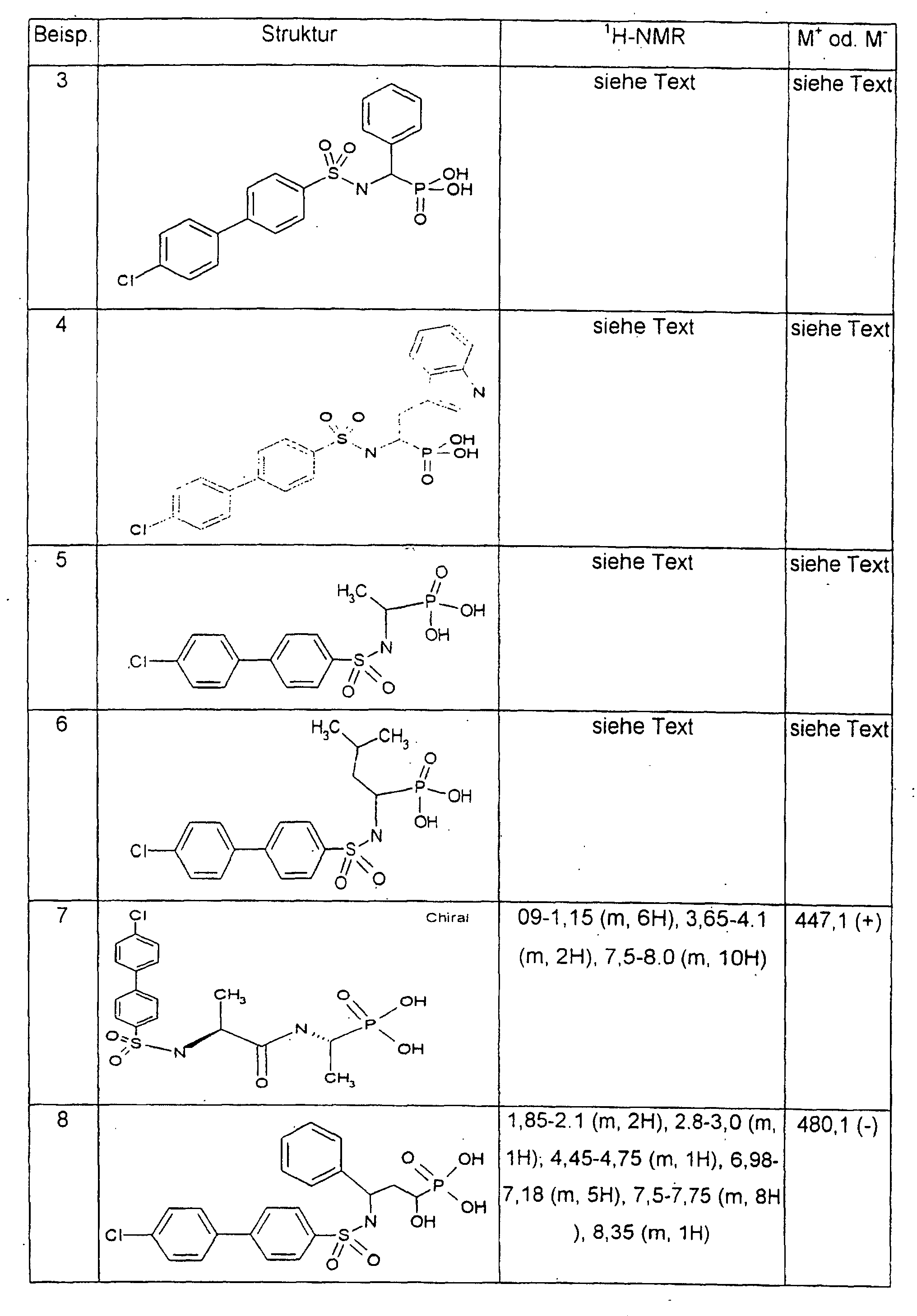

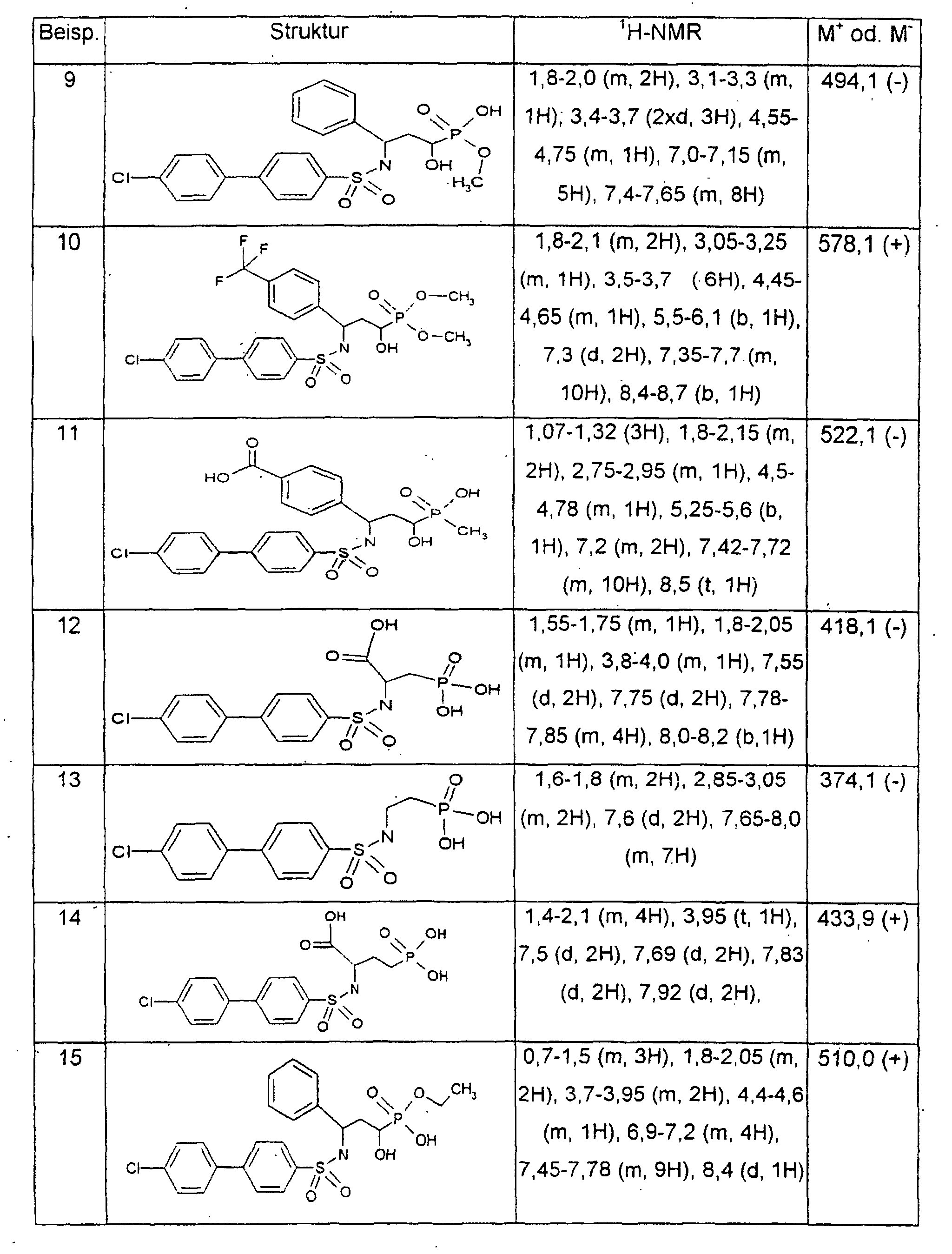

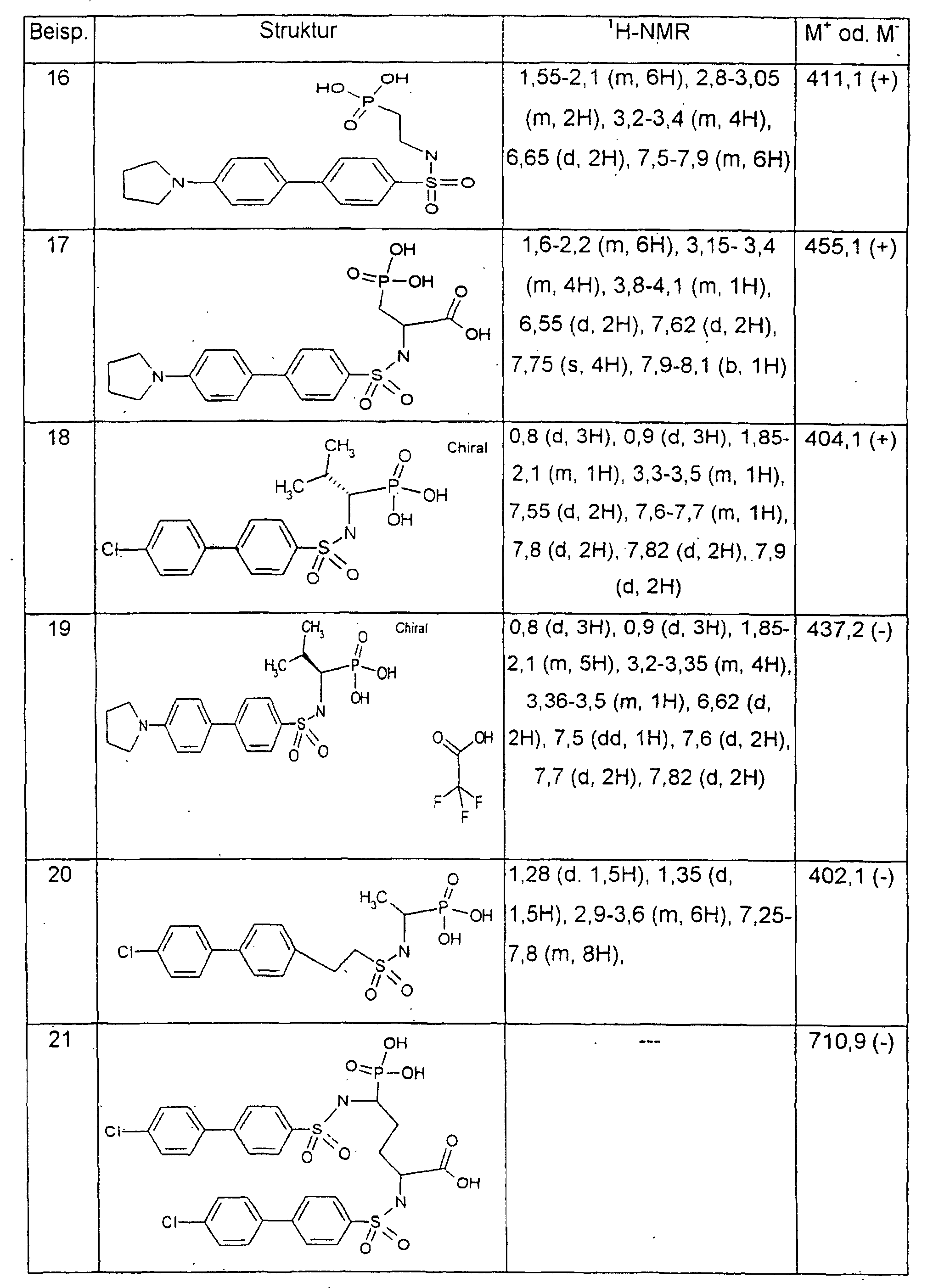

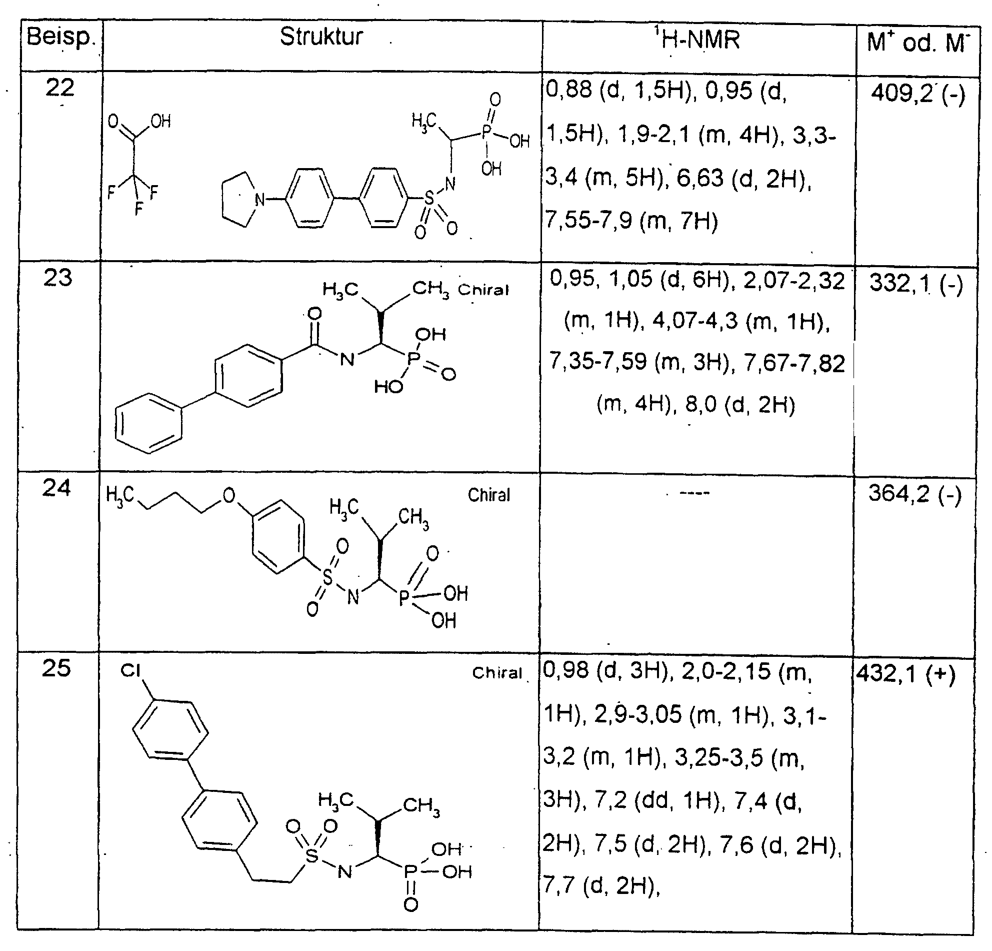

### Pharmakologische Beispiele

Darstellung und Bestimmung der enzymatischen Aktivität der katalytischen Domäne des humanen Stromelysins und der Neutrophilen-Kollagenase.

Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung werden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthält, für 15 Minuten inkubiert. Nach Zugabe von 10 µl einer 10%igen (v/v) wäßrigen Dimethylsulfoxid-Lösung, die 1 mmol/l des Substrates enthält, wird die Enzymreaktion fluoreszenzspektroskopisch verfolgt (328 nm (ex) / 393 nm(em)).

Die Enzymaktivität wird dargestellt als Extinktionszunahme/Minute. Die in Tabelle 2 aufgeführten IC₅₀-Werte werden als diejenige Inhibitorkonzentrationen ermittelt, die jeweils zu einer 50%igen Inhibierung des Enzyms führen.
Die Pufferlösung enthält 0,05% Brij (Sigma, Deisenhofen, Deutschland) sowie 0,1 mol/l Tris/HCl, 0,1 mol/l NaCl, 0,01 mol/l CaCl₂ und 0,1 mol/l Piperazin-N,N'-bis[2-ethansulfonsäure] (pH=6,5).
Die Enzymlösung enthält 5 µg/ml einer der nach Ye et al. dargestellten Enzymdomänen. Die Substratlösung enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland)

**Tabelle 2**

| **Beispiel Nr.** | **Stromelysin IC**_{**50**} **(M)** | **Neutr. Kollagenase IC**_{**50**} **(M)** |
|---|---|---|
| 1 | 6*10⁻⁹ | 1*10⁻⁹ |
| 2 | 5*10⁻⁶ | 2*10⁻⁷ |
| 3 | 1*10⁻⁷ | 7*10⁻⁹ |
| 4 | 5*10⁻⁷ | 6*10⁻⁸ |
| 5 | 1*10⁻⁷ | 5*10⁻⁹ |
| 6 | 3*10⁻⁸ | 3*10⁻⁹ |
| 18 | 10*10⁻⁶ | 1*10⁻⁶ |
| 19 | 5*10⁻⁹ | 2*10⁻⁹ |
| 20 | 8*10⁻⁹ | 2*10⁻⁹ |
| 22 | 2*10⁻⁸ | 2*10⁻⁸ |
| 24 | 4*10⁻⁷ | 3*10⁻⁸ |
| 25 | 3*10⁻⁹ | 2*10⁻⁹ |

Darstellung und Bestimmung der enzymatischen Aktivität der katalytischen Domäne der Aggrecanase mit Rattenchondrosarcomzellen
Zur Generierung der bisher noch nicht identifizierten "Aggrecanase"-Aktivität wurden Rattenchondrosarcomzellen (RCS) verwendet (Lark et al.; J. Biol. Chem., 270; (1995), 2550-2556). Diese Zellen wurden auf mit Poly-L-Lysin vorbeschichteten 96 Well Zellkulturplatten ausgesät (80000 Zellen/Well). Nach einer Stimulation der RCS-Zellen mit Retinsäure (0,67 µM) und einer inkubationszeit von 47 Stunden (h) bei 37°C und 5% CO₂ generieren diese Zellen die "Aggrecanase"-Aktivität. Die Testsubstanz Verbindung 1 wurde daraufhin für 1h in dem "Aggrecanase"-haltigen Zellkulturüberstand vorinkubiert, bevor zum Nachweis der "Aggrecanase"-Spaltungsaktivität in den Zellkulturüberstand der RCS-Zellen 5µg eukaryontisches rAgg1ₘᵤₜ (Büttner et al., Biochem. J. 333; (1998), 159-165 und Hughes et al., J. Biol. Chem. 272; (1997), 20269-20274) gegeben wurden. Nach einer Inkubationszeit von 4h wurde der Zellkulturüberstand abgenommen und die durch die "Aggrecanase"-Aktivität generierten Spaltprodukte der rAgg1ₘᵤₜ-Fusionsproteine mittels SDS-Polyacrylamid-Gelelektrophorese und Western-Blot Analysen mit dem monoklonalen Antikörper BC-3 (Hughes et al. Biochem. J. 305, 799-804, 1995) detektiert. Die Wirkung der Verbindung 1 zeigte sich in der Verringerung der BC-3 reaktiven Spaltprodukte.Je weniger gespaltenes rAgg1ₘᵤₜ detektiert wurde, desto wirksamer war die getestete Verbindung der Formel I.

Die in Tabelle 3 aufgeführten IC₅₀-Werte werden als diejenige Inhibitorkonzentrationen ermittelt, die jeweils zu einer 50%igen Inhibierung des Enzyms Aggrecanase führten.

**Tabelle 3**

| **Beispiel Nr.** | **Aggrecanase IC**_{**50**} |
|---|---|
| 1 | 0,6 µM |
| 2 | 79 µM |
| 3 | 22 µM |
| 4 | 12 µM |
| 5 | 25 µM |
| 6 | 2,4 µM |
| 7 | 29 µM |
| 8 | 15 µM |
| 10 | 2,1 µM |
| 12 | 50 µM |
| 13 | 50 µM |
| 14 | 55 µM |
| 15 | 67 µM |
| 16 | 28 µM |
| 17 | 69 µM |
| 18 | 60 µM |
| 20 | 4,7 µM |
| 21 | 0,52 µM |
| 22 | 8,3 µM |

## Patentansprüche

1. Verbindung der Formel I 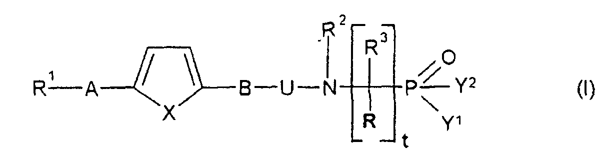 und/oder eine stereoisomere Form der Verbindung der Formel I und/oder ein physiologisch verträgliches Salz der Verbindung der Formel I, wobei
R¹ für
1. Phenyl,
2. Phenyl, welches ein- oder zweifach substituiert ist durch
2.1. (C₁-C₆)-Alkyl, gerade, cyclisch oder verzweigt,
2.2. Hydroxy,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁴-(R⁵)N-C(O)-,
2.14. R⁴-(R⁵ )N- oder
2.15 Heteroaromaten aus der Gruppe 3.1. bis 3.16,
3. einen Heteroaromaten aus der nachfolgenden Gruppe 3.1. bis 3.16, der unsubstituiert oder wie unter 2.1 bis 2.15 beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Pyrrolidin,
3.12. Indol,
3.13. Benzothiophen,
3.14. Benzimidazol,
3.15. Benzoxazol oder
3.16. Benzothiazol, oder
4. -O-(C₁-C₆)-Alkyl, steht,
R², R⁴ und R⁵ gleich oder verschieden sind und für
1. Wasserstoffatom,
2. (C₁-C₆)-Alkyl-,
3. HO-C(O)-(C₁-C₆)-Alkyl-,
4. Phenyl-(CH₂)ₙ-, worin Phenyl unsubstituiert oder ein- oder zweifach wie unter 2.1. bis 2.15. beschrieben substituiert oder durch -NH-C(O)-(C₁-C₃)-Alkyl substituiert ist und n die ganze Zahl Null, 1 oder 2 darstellt, oder
5. Picolyl stehen oder
6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist oder zwei benachbarte C-Atome des 4- bis 7-gliedrigen Rings Teil eines Benzylrestes sind,
R und R³ gleich oder verschieden sind und für
1. Wasserstoffatom,
2. (C₁-C₁₀)-Alkyl-, worin Alkyl unsubstituiert ist oder ein Wasserstoffatom des Alkylrestes durch -OH ersetzt ist,
3. (C₂-C₁₀)-Alkenyl-, worin Alkenyl gerade oder verzweigt ist,
4. R²-O-(C₁-C₆)-Alkyl-,
5. R²-S(O)ₙ-(C₁-C₆)-Alkyl-, wobei n die obengenannte Bedeutung hat,
6. R²-S(O)(-NH)-(C₁-C₆)-Alkyl-,
7. Rest der Formel IIo steht, 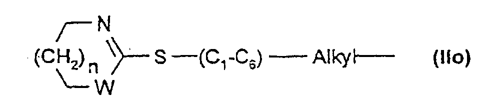 worin n die ganze Zahl Null, 1 oder 2 darstellt und W ein Stickstoff-, Sauerstoff- oder Schwefelatom darstellt,
8. Phenyl-(CH₂)ₘ-, worin m die ganze Zahl Null, 1, 2, 3, 4, 5 oder 6 ist und/oder ein Wasserstoffatom der -(CH₂)ₘ-Kette durch -OH ersetzt ist und Phenyl unsubstituiert oder ein- oder zweifach substituiert ist mit
8.1 wie unter 2.1. bis 2.15. beschrieben,
8.2 -O-(CH₂)ₘ-Phenyl, worin Phenyl unsubstituiert oder ein- oder zweifach wie unter 2.1. bis 2.15. beschrieben substituiert ist und m die ganze Zahl Null, 1, 2, 3, 4, 5 oder 6 darstellt,
8.3 -C(O)-(CH₂)ₘ-Phenyl, worin Phenyl wie unter 8.2 definiert ist,
9. Heteroaryl-(CH₂)ₘ-, worin Heteroaryl wie unter 3.1. bis 3.16. definiert ist, m wie oben definiert ist und/oder ein Wasserstoffatom der -(CH₂)ₘ-Kette durch -OH ersetzt ist und Heteroaryl unsubstituiert oder ein- oder zweifach substituiert ist mit
9.1 wie unter 2.1 bis 2.15 beschrieben,
9.2 -CH(O),
9.3 -SO₂-Phenyl, worin Phenyl unsubstituiert oder wie unter 8.2 definiert ist,
9.4 -O-(CH₂)ₘ-Phenyl,
10. -(CH₂)ₘ-P(O)(OH)-(C₁-C₃)-Alkyl, worin m wie oben definiert ist.
11. charakteristischer Rest einer Aminosäure oder
12. R⁶-C(O)-(C₀-C₆)-Alkyl- steht, worin R⁶ für
1. Wasserstoffatom,
2. (C₁-C₆)-Alkyl-, worin Alkyl gerade, verzweigt oder cyclisch ist,
3. Phenyl, worin Phenyl unsubstituiert oder wie unter 2.1 bis 2.15 beschrieben substituiert ist,
4. Heteroaryl, worin Heteroaryl wie unter 3.1. bis 3.16. definiert und/oder wie unter 2.1 bis 2.15 beschrieben substituiert oder durch -(C₁-C₄)-Alkyl-COOH substituiert ist,
5. -OH,
6. -OR², worin R² die obengenannte Bedeutung hat,
7. -NR⁴-(R⁵) bedeutet, worin R⁴ und R⁵ wie oben definiert sind,
8. Heteroaryl-(CH₂)ₘ-NH-, worin Heteroaryl wie unter 3.1 bis 3.16. definiert und/oder wie unter 2.1. bis 2.15 beschrieben substituiert ist und m wie oben definiert ist,
9. R⁴-(R⁵)N-NH-, worin R⁴ und R⁵ wie oben definiert sind,
10. HO-C(O)-CH(R³)-NH-, worin R³ wie oben definiert ist,
13. -(CH₂)ₚ-N(R⁹)(R¹⁰), worin p eine ganze Zahl Null, 1,2, 3 oder 4 darstellt, worin R⁹ und R¹⁰ gleich oder verschieden sind und für
1. Wasserstoffatom,
2. Phenyl-(CH₂)ₘ-, worin Phenyl unsubstituiert oder ein- oder zweifach wie unter 2.1. bis 2.15. beschrieben substituiert ist und m die ganze Zahl Null, 1, 2 oder 3 ist,
3. R^{x}-C(O)-, worin R^{x} für
3.1 (C₁-C₆)-Alkyl-,
3.2 (C₂-C₆)-Alkenyl-,
3.3 Phenyl-(CH₂)ₘ-, worin Phenyl unsubstituiert oder ein- oder zweifach wie unter 2.1. bis 2.15. beschrieben substituiert ist und m die ganze Zahl Null, 1, 2 oder 3 darstellt, oder
3.4 Heteroaryl-(CH₂)ₘ-, worin Heteroaryl wie unter 3.1.bis 3.16. definiert und/oder wie unter 2.1 bis 2.15 beschrieben substituiert ist und m die ganze Zahl Null, 1, 2 oder 3 darstellt, steht,
4. R^{x}-O-C(O)-, worin R^{x} wie oben genannt definiert ist,
5. R^{x}-CH(NH₂)-C(O)-, worin R^{x} wie oben genannt definiert ist,
6. R⁸-N(R⁷)-C(O)-, worin R⁸ für
6.1 Wasserstoffatom
6.2 (C₁-C₆)-Alkyl-,
6.3 Phenyl-(CH₂)ₘ, worin Phenyl unsubstituiert oder ein- oder zweifach wie unter 2.1. bis 2.15. beschrieben substituiert ist und m die ganze Zahl Null, 1,2 oder 3 darstellt, oder
6.4 Heteroaryl-(CH₂)ₘ, worin Heteroaryl wie unter 3.1. bis 3.16. definiert und/oder wie unter 2.1 bis 2.15 beschrieben substituiert ist und m die ganze Zahl Null, 1, 2 oder 3 darstellt, steht und worin R⁷ Wasserstoffatom oder (C₁-C₆)-Alkyl- bedeutet oder worin R⁷ und R⁸ zusammen mit dem Stickstoffatom an das sie gebunden sind einen 4- bis 7-gliedrigen Ring bilden und der Ring unsubstituiert ist oder ein Kohlenstoffatom im Ring durch -O-, -S- oder -NH- ersetzt ist,
7. R^{x}-SO₂-, worin R^{x} wie oben genannt definiert ist,
8. R^{x}-NH-C(=NR⁷)-, worin R^{x} und R⁷ wie oben genannt definiert sind oder für
8.1. (C₁-C₆)-Alkyl-C(O)-,
8.2 -NO₂ oder
8.3 -SO₂-(CH₂)_{q}-Phenyl, worin Phenyl unsubstituiert oder ein- oder zweifach wie unter 2.1. bis 2.15. beschrieben substituiert ist und q die ganze Zahl Null, 1,2 oder 3 ist, stehen,
9. -SO₂-(CH₂)_{q}-Phenyl-Phenyl, worin Phenyl unsubstituiert oder einoder zweifach wie unter 2.1. bis 2.15. beschrieben substituiert ist und q die ganze Zahl Null, 1,2 oder 3 ist, darstellen, oder
10. den Rest der Formel llp steht, 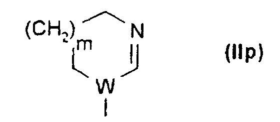 worin m die ganze Zahl Null, 1, 2 oder 3 bedeutet und W für ein Stickstoffatom steht, oder
R⁹ und R¹⁰ zusammen mit dem Stickstoffatom an das sie gebunden sind einen Ring der Teilformel IIa bis IIm bilden, 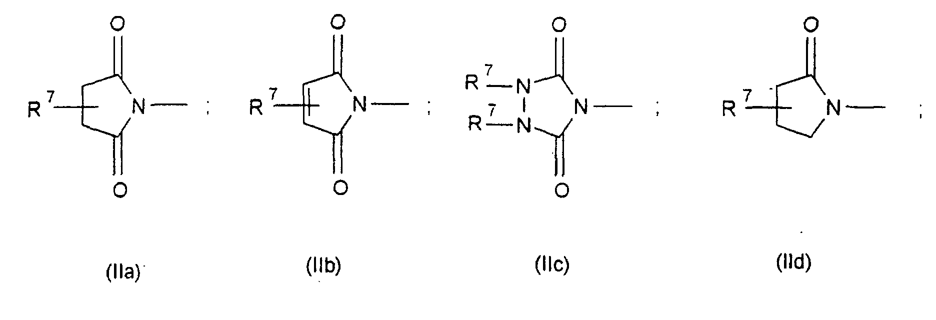 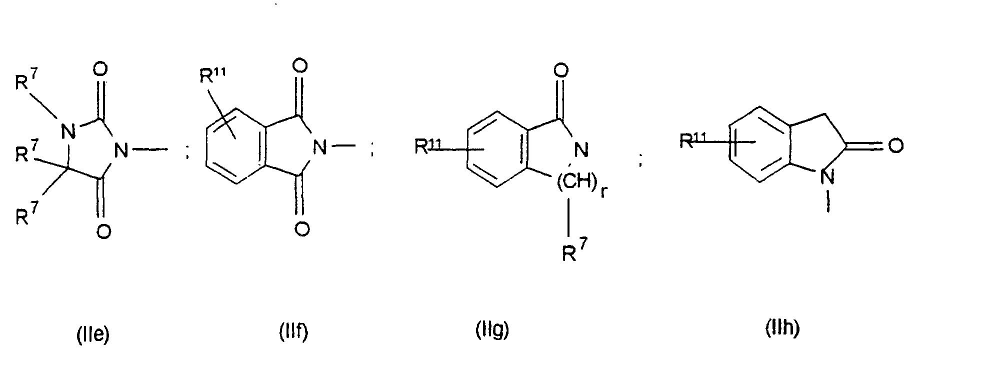 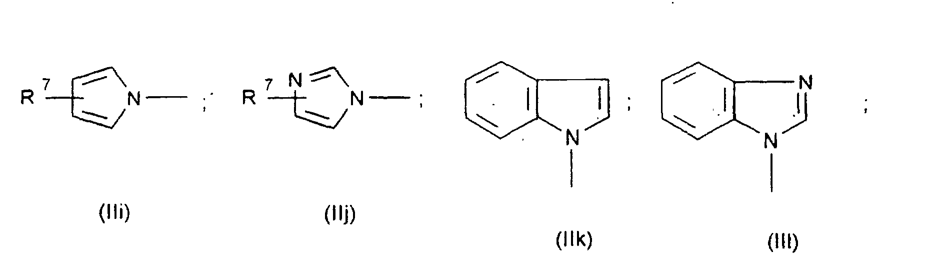 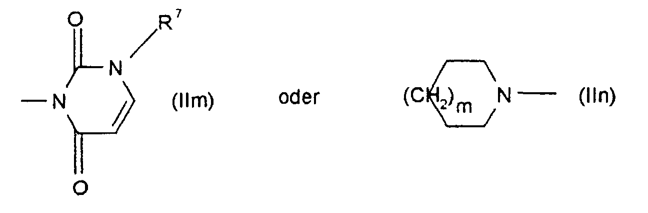 wobei r die ganze Zahl 1 oder 2 bedeutet, R¹¹ einen Rest wie unter 2.1. bis 2.15 beschrieben bedeutet, und R⁷ und m die oben genannte Bedeutung haben,
14. -OH,
15. =O oder
16. (C₁-C₆)-Alkyl- steht, oder ein -C(R)(R³)- Rest für, -NH- oder -NR²- steht, worin R² wie oben definiert ist, und
t für eine ganze Zahl 1, 2, 3 oder 4 steht, oder
R² und R³ bilden zusammen einen Ring mit exocyclischem Phosphin- oder Phosphonsäurerest der Teilformel II, 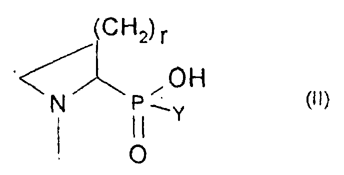 worin r die ganze Zahl Null, 1, 2 oder 3 darstellt und/oder eines der Kohlenstoffatome im Ring durch -O-, -S- oder -(R⁷)N- ersetzt ist, worin
R⁷ für
1. Wasserstoffatom,
2. (C₁-C₆)-Alkyl,
3. Phenyl, worin Phenyl unsubstituiert oder wie unter 2.1 bis 2.15 beschrieben substituiert ist,
4. Benzyl, worin Benzyl unsubstituiert oder wie unter 2.1 bis 2.15 beschrieben substituiert ist, oder
5. R²N-C(=NH)- steht, wobei R² die obengenannte Bedeutung hat, und/oder die Kohlenstoffatome im Ring der Teilformel II ein- oder mehrfach durch (C₁-C₆)-Alkyl-, Phenyl-, Phenyl-(CH₂)ₘ- oder HO- substituiert sind,
U für -SO₂- oder -CO- steht,
Y¹ und Y² gleich oder verschieden sind und unabhängig voneinander für
a) Wasserstoffatom,
b) -OH,
c) -(C₁-C₄)-Alkyl, worin Alkyl gerade oder verzweigt ist,
d) -(CH₂)ᵤ-Phenyl, worin u Null oder 1 ist,
e) -O-(C₁-C₄)-Alkyl, worin Alkyl gerade oder verzweigt ist, oder
f) -O-(CH₂)ₛ-Phenyl, worin s Null oder 1 ist, steht,
A für
a) eine kovalente Bindung,
b) -O-,
c) -CH=CH- oder
d) -C=C- steht,
B für
a) -(CH₂)ₒ-, worin o die ganze Zahl Null, 1, 2, 3 oder 4 bedeutet,
b) -O-(CH₂)ₚ, worin p eine ganze Zahl von 1 bis 5 bedeutet, oder
c) -CH=CH- steht, und
X für -CH=CH-, Sauerstoffatom oder Schwefelatom steht.

2. Verbindung der Formel I gemäß Anspruch 1, **dadurch gekennzeichnet, daß**
R¹ für
1. Phenyl oder
2. Phenyl, welches einfach substituiert ist durch
2.1. (C₁-C₆)-Alkyl-, worin Alkyl gerade, cyclisch oder verzweigt ist,
2.2. -OH,
2.3. -C(O)-OH,
2.4. -O-(C₁-C₆)-Alkyl,
2.5. Pyrrolidin,
2.6. Halogen oder
2.7. -CF₃ steht, oder
3. -O-(C₁-C₆)-Alkyl, steht
R², R⁴ und R⁵ gleich oder verschieden sind und für Wasserstoffatom oder (C₁-C₆)-Alkyl- stehen,
R für Wasserstoffatom steht,
R³ für
1. (C₁-C₆)-Alkyl-, worin Alkyl gerade, verzweigt oder cyclisch ist, und/oder ein Wasserstoffatom des Alkylrestes durch -OH ersetzt ist,
2. R²-S(O)ₙ-(C₁-C₆)-Alkyl-, worin R² (C₁-C₆)-Alkyl- oder Phenyl-(CH₂)ₙ- bedeutet und n die ganze Zahl Null oder 1 ist,
3. -(CH₂)ₘ-Phenyl, worin Phenyl unsubstituiert oder ein- oder zweifach wie unter 2.1. bis 2.15. beschrieben substituiert ist und/oder ein Wasserstoffatom der -(CH₂)ₘ- Kette durch -OH ersetzt ist und m die ganze Zahl 1, 2, 3, 4 oder 5 darstellt,
4. -(CH₂)ₘ-Heteroaryl, worin Heteroaryl wie unter 3.3, 3.5, 3.6, 3.9 oder 3.11 definiert ist und/oder wie unter 2.1. bis 2.15 beschrieben substituiert ist und/oder ein Wasserstoffatom der -(CH₂)ₘ-Kette durch -OH ersetzt ist und m die ganze Zahl 1, 2, 3 oder 4 ist,
5. charakteristischer Rest einer Aminosäure oder
6. -(CH₂)ₚ-N(R⁹)(R¹⁰), worin p eine ganze Zahl Null, 1, oder 2 darstellt, worin R⁹ und R¹⁰ gleich oder verschieden sind und für Wasserstoffatom oder -SO₂-(CH₂)_{q}-Phenyl-Phenyl, worin Phenyl unsubstituiert oder ein- oder zweifach wie unter 2.1. bis 2.15. beschrieben substituiert ist und q die ganze Zahl Null, 1,2 oder 3 ist, darstellen, steht oder
7. R⁶-C(O)- steht, worin R⁶ für
7.1. -OH,
7.2. R²O-, worin R² wie oben definiert ist, oder
7.3. R⁴-(R⁵)N- steht, worin R⁴ und R⁵ wie oben definiert sind,
8. Wasserstoffatom,
9. -OH,
10. =O oder
11. (C₁-C₆)-Alkyl steht, oder
ein -C(R)(R³)- Rest für, -NH- oder -NR²- steht, worin R² wie oben definiert ist, und
t für eine ganze Zahl 1, 2, 3 oder 4 steht,
U für -SO₂- steht,
Y¹ für -OH steht,
Y² für
a) -O-(C₁-C₄)-Alkyl, worin Alkyl gerade oder verzweigt ist,
b) -OH oder
c) -(C₁-C₄)-Alkyl, worin Alkyl gerade oder verzweigt ist, steht,
A für eine kovalente Bindung oder -O- steht,
B für eine kovalente Bindung oder -(C₁-C₄)-Alkyl steht und
X für -CH=CH- steht.

3. Verbindung der Formel I, gemäß Anspruch 1 oder 2, **dadurch gekennzeichnet, daß**
R¹ für 1. Phenyl steht, welches einfach durch Halogen substituiert ist,
R² für Wasserstoffatom steht,
R für Wasserstoffatom steht,
R³ für
1. (C₁-C₄)-Alkyl-,
2. -Phenyl, worin Phenyl unsubstituiert oder ein- oder zweifach durch - CF₃ oder -COOH substituiert ist,
3. Wasserstoffatom,
4. -OH oder
5. -NH-SO₂-Phenyl-Phenyl, worin Phenyl unsubstituiert oder durch Halogen substituiert ist, steht,
t für eine ganze Zahl 1, 2, 3 oder 4 steht,
U für -SO₂-steht,
Y¹ und Y² für -OH oder -O-CH₃ steht,
A für eine kovalente Bindung steht,
B für eine kovalente Bindung oder -(CH₂)ₒ- steht, worin o 1, 2 oder 3 bedeutet und
X für -CH=CH- steht.

4. Verbindung ( R )- [1-(4'-Chlorbiphenyl-4-sulfonyl-amino)-2-methylpropyl]-phosphonsäure, [3-(4'-Chlorbiphenyl-4-sulfonylamino)-1-hydroxy-3-(4-trifluormethyl-phenyl)-propyl]-phosphonsäuredimethylester, [1-(4'-Chlor-biphenyl-4-sulfonylamino)-3-methyl-butyl]-phosphonsäure oder ( R,S )- [1-(4'-Chlor-biphenyl-4-sulfonylamino)-1 -phenylmethyl]-phosphonsäure-monoethylester.

5. Verbindung der Formel VI 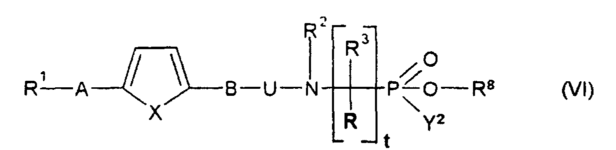 und/oder eine stereoisomere Form der Verbindung der Formel VI und/oder ein physiologisch verträgliches Salz der Verbindung der Formel VI, wobei R¹, A, X, B, U, Y², t, R²und R³ die in der Verbindung der Formel I gemäß Anspruch 1 genannte Bedeutung haben und R⁸ die in der Verbindung der Formel IV gemäß Anspruch 6 genannte Bedeutung hat.

6. Verfahren zur Herstellung der Verbindung der Formel I gemäß einem oder mehreren der Ansprüche 1 bis 5, **dadurch gekennzeichnet, daß** man
a) eine Aminophosphin- oder phosphonsäure der Formel III, 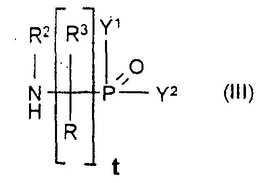 worin R², Y¹, Y², R und R³ wie in Formel I definiert sind, mit einem Sulfonsäure-oder Carbonylderivat der Formel IV, 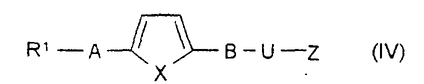 worin R¹, A , X, U und B wie in Formel I definiert sind und Z ein Halogenatom, Imidazolyl oder -OR⁸ bedeutet, worin R⁸ Wasserstoffatom, (C₁-C₆)-Alkyl, Phenyl-oder Benzyl, gegebenenfalls substituiert darstellt,
in Gegenwart einer Base oder gegebenenfalls eines wasserentziehenden Mittels zu einer Verbindung der Formel I umsetzt, oder
b) einen Aminophosin- oder phosphonsäureester der Formel V, 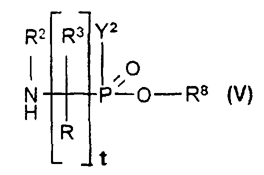 worin R², R³, t, Y² und R⁸ die obengenannte Bedeutung haben,
mit einem Sulfonsäure- oder Carbonylderivat der Formel IV zu einer Verbindung der Formel VI 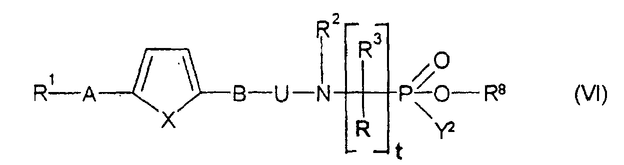 umsetzt, und die Verbindung der Formel VI unter Abspaltung des Restes R⁸, bevorzugt in Gegenwart einer Base oder Säure in eine Verbindung der Formel I umwandelt, oder
c) die Verbindung der Formel VII, 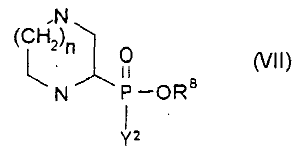 wobei n die ganze Zahl Null, 1 oder 2 darstellt,
mit Hilfe einer Schutzgruppe E zu einer Verbindung der Formel VIII umsetzt, 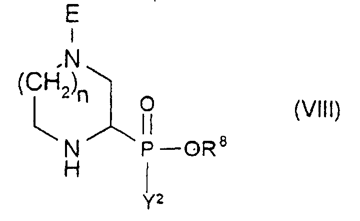 und die Verbindung der Formel VIII mit einer Verbindung der Formel IV in eine Verbindung der Formel IX überführt, 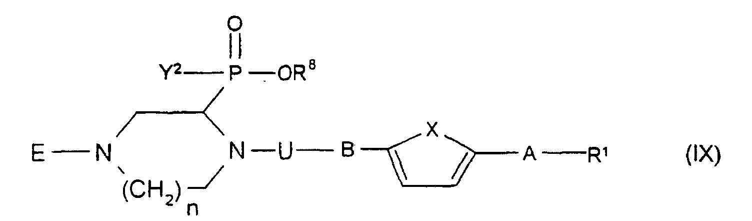 und anschließend die Verbindung der Formel IX unter Abspaltung der Schutzgruppe E und des Restes R⁸ mit Hilfe geeigneter Spaltreagenzien in die Verbindung der Formel I überführt, oder
d) eine nach den Verfahren a), b) oder c) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
e) die nach den Verfahren a), b), c) oder d) hergestellte Verbindung der Formel I entweder in freier Form isoliert oder im Falle des Vorliegens von sauren oder basischen Gruppen in physiologisch verträgliche Salze umwandelt.

7. Arzneimittel, **gekennzeichnet durch** einen wirksamen Gehalt an mindestens einer Verbindung der Formel I gemäß einem oder mehreren der Ansprüche 1 bis 5 zusammen mit einem pharmazeutisch geeigneten und physiologisch verträglichen Trägerstoff, Zusatzstoff und/oder anderen Wirk- und Hilfsstoffen.

8. Verwendung von mindestens einer Verbindung der Formel I gemäß einem oder mehreren der Ansprüche 1 bis 5, zur Herstellung von Arzneimitteln zur Prophylaxe und Therapie von Erkrankungen, an deren Verlauf eine verstärkte Aktivität von Matrix-abbauenden Metalloproteinasen beteiligt ist.

9. Verwendung gemäß Anspruch 8, zur Behandlung von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, der Ulceration, Atherosklerose und Stenosen, aber auch zur Behandlung von Entzündungen, Krebserkrankungen, Tumormetastasenbildung, Kachexie, Anorexie und septischem Schock.

10. Verfahren zur Herstellung eines Arzneimittels, **dadurch gekennzeichnet, daß** man mindestens eine Verbindung der Formel I gemäß einem oder mehreren der Ansprüche 1 bis 5 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 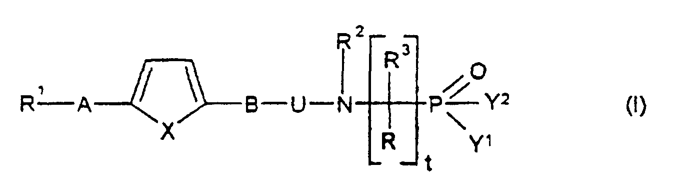 and/or a stereoisomeric form of the compound of the formula I and/or a physiologically acceptable salt of the compound of the formula I, where
R¹ is
1. phenyl,
2. phenyl which is mono- or disubstituted by
2.1. (C₁-C₆)-alkyl, which is linear, cyclic or branched,
2.2. hydroxyl,
2.3. (C₁-C₆)-alkyl-C(O)-O-,
2.4. (C₁-C₆)-alkyl-O-,
2.5. (C₁-C₆)-alkyl-C-(C₁-C₄)-alkyl-O-,
2.6. halogen,
2.7. -CF₃,
2.8. -CN,
2.9. -NO₂,
2.10. HO-C(O)-,
2.11. (C₁-C₆)-alkyl-O-C(O)-,
2.12. methylenedioxo,
2.13. R⁴-(R⁵)N-C(O)-,
2.14. R⁴-(R⁵)N- or
2.15. heteroaromatics from the group 3.1. to 3.16.,
3. a heteroaromatic from the following group 3.1. to 3.16., which is unsubstituted or substituted as described under 2.1. to 2.15.,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. pyrrolidine,
3.12. indole,
3.13. benzothiophene,
3.14. benzimidazole,
3.15. benzoxazole or
3.16. benzothiazole, or
4. -O-(C₁-C₆)-alkyl,
R², R⁴ and R⁵ are identical or different and are
1. a hydrogen atom,
2. (C₁-C₆)-alkyl-,
3. HO-C(O)-(C₁-C₆)-alkyl-,
4. phenyl-(CH₂)ₙ-, in which phenyl is unsubstituted or mono- or disubstituted as described under 2.1. to 2.15. or is substituted by -NH-C(O)-(C₁-C₃)-alkyl and n is the integer zero, 1 or 2, or
5. picolyl or
6. R⁴ and R⁵ together with the ring amino group form a 4- to 7-membered ring in which one of the carbon atoms is optionally replaced by -O-, -S- or -NH- or two adjacent carbon atoms of the 4- to 7-membered ring are part of a benzyl radical,
R and R³ are identical or different and are
1. a hydrogen atom,
2. (C₁-C₁₀)-alkyl-, in which alkyl is unsubstituted, and/or a hydrogen atom of the alkyl radical is replaced by -OH,
3. (C₂-C₁₀)-alkenyl-, in which alkenyl is linear or branched,
4. R²-O-(C₁-C₆)-alkyl-,
5. R²-S(O)ₙ-(C₁-C₆)-alkyl-, where n has the abovementioned meaning,
6. R²-S(O)(=NH)-(C₁-C₆)-alkyl-,
7. a radical of the formula IIo 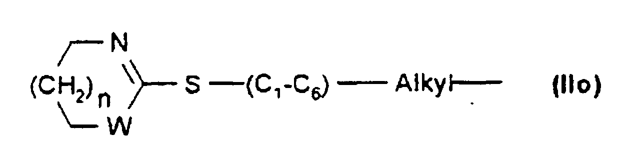 in which n is the integer zero, 1 or 2 and W is a nitrogen, oxygen or sulfur atom,
8. phenyl-(CH₂)ₘ-, in which m is the integer zero, 1, 2, 3, 4, 5 or 6 and/or a hydrogen atom of the -(CH₂)ₘ- chain is replaced by -OH and phenyl is unsubstituted or mono- or disubstituted by
8.1. as described under 2.1. to 2.15.,
8.2. -O-(CH₂)ₘ-phenyl, in which phenyl is unsubstituted or mono- or disubstituted as described under 2.1. to 2.15. and
m is the integer zero, 1, 2, 3, 4, 5 or 6,
8.3. -C(O)-(CH₂)ₘ-phenyl, in which phenyl is as defined under 8.2.,
9. heteroaryl-(CH₂)ₘ-, in which heteroaryl is as defined under 3.1. to 3.16., m is as defined above and/or a hydrogen atom of the -(CH₂)ₘ- chain is replaced by -OH and heteroaryl is unsubstituted or mono- or disubstituted by
9.1. as described under 2.1. to 2.15.,
9.2. -CH(O),
9.3. -SO₂-phenyl, in which phenyl is unsubstituted or as defined under 8.2. or 8.3.,
9.4. -O-(CH₂)ₘ-phenyl,
10. -(CH₂)ₘ-P(O)(OH)-(C₁-C₃)-alkyl, in which m is as defined above,
11. a characteristic residue of an amino acid or
12. R⁶-C(O)-(C₀-C₆)-alkyl-, in which R⁶ is
1. a hydrogen atom,
2. (C₁-C₆)-alkyl-, in which alkyl is linear, branched or cyclic,
3. phenyl, in which phenyl is unsubstituted or substituted as described under 2.1. to 2.15.,
4. heteroaryl, in which heteroaryl is as defined under 3.1. to 3.16. and/or is substituted as described under 2.1. to 2.15. or substituted by -(C₁-C₄)-alkyl-COOH,
5. -OH,
6. -OR², in which R² has the abovementioned meaning,
7. -NR⁴-(R⁵), in which R⁴ and R⁵ are as defined above,
8. heteroaryl-(CH₂)ₘ-NH-, in which heteroaryl is as defined under 3.1. to 3.16. and/or is substituted as described under 2.1. to 2.15. and m is as defined above,
9. R⁴-(R⁵)N-NH-, in which R⁴ and R⁵ are as defined above,
10. HO-C(O)-CH(R³)-NH-, in which R³ is as defined above,
13. -(CH₂)ₚ-N(R⁹)(R¹⁰), in which p is an integer zero, 1, 2, 3 or 4, in which R⁹ and R¹⁰ are identical or different and are
1. a hydrogen atom,
2. phenyl-(CH₂)ₘ-, in which phenyl is unsubstituted or mono- or disubstituted as described under 2.1. to 2.15. and m is the integer zero, 1, 2 or 3,
3. R^{x}-C(O)-, in which R^{x} is
3.1. (C₁-C₆)-alkyl-,
3.2. (C₂-C₆)-alkenyl-,
3.3. phenyl-(CH₂)ₘ-, in which phenyl is unsubstituted or mono- or disubstituted as described under 2.1. to 2.15. and m is the integer zero, 1, 2 or 3, or
3.4. heteroaryl-(CH₂)ₘ-_{,} in which heteroaryl is as defined under 3.1. to 3.16. and/or is substituted as described under 2.1. to 2.15. and m is the integer zero, 1, 2 or 3,
4. R^{x}-O-C(O)-, in which R^{x} is defined as mentioned above,
5. R^{x}-CH(NH₂)-C(O)-, in which R^{x} is defined as mentioned above,
6. R⁸-N(R⁷)-C(O)-, in which R⁸ is
6.1. a hydrogen atom,
6.2. (C₁-C₆)-alkyl-,
6.3. phenyl-(CH₂)ₘ-, in which phenyl is unsubstituted or mono- or disubstituted as described under 2.1. to 2.15. and m is the integer zero, 1, 2 or 3, or
6.4. heteroaryl-(CH₂)ₘ-, in which heteroaryl is as defined under 3.1. to 3.16. and/or is substituted as described under 2.1. to 2.15. and m is the integer zero, 1, 2 or 3 and in which R⁷ is a hydrogen atom or (C₁-C₆)-alkyl- or in which R⁷ and R⁸ together with the nitrogen atom to which they are attached form a 4- to 7-membered ring and the ring is unsubstituted or a carbon atom in the ring is replaced by -O-, -S- or -NH-,
7. R^{x}-SO₂-, in which R^{x} is defined as mentioned above,
8. R^{x}-NH-C(=NR⁷)-, in which R^{x} and R⁷ are defined as mentioned above or are
8.1. (C₁-C₆)-alkyl-C(O)-,
8.2. -NO₂ or
8.3. -SO₂-(CH₂)_{q}-phenyl, in which phenyl is unsubstituted or mono- or disubstituted as described under 2.1. to 2.15. and q is the integer zero, 1, 2 or 3,
9. -SO₂-(CH₂)_{q}-phenyl-phenyl, in which phenyl is unsubstituted or mono- or disubstituted as described under 2.1. to 2.15. and q is the integer zero, 1, 2 or 3, or
10. the radical of the formula IIp 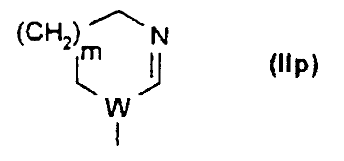 in which m is the integer zero, 1, 2 or 3 and W is a nitrogen atom, or
R⁹ and R ¹⁰ together with the nitrogen atom to which they are attached form a ring of the subformula IIa to IIm 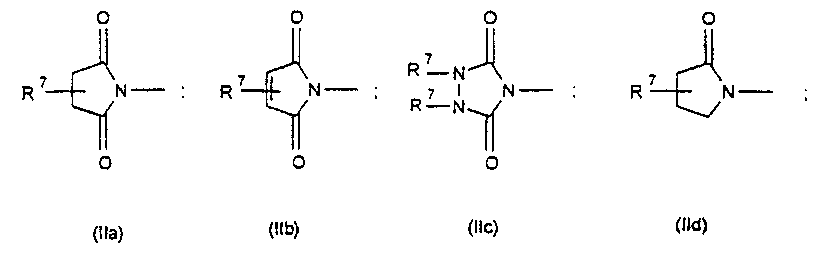 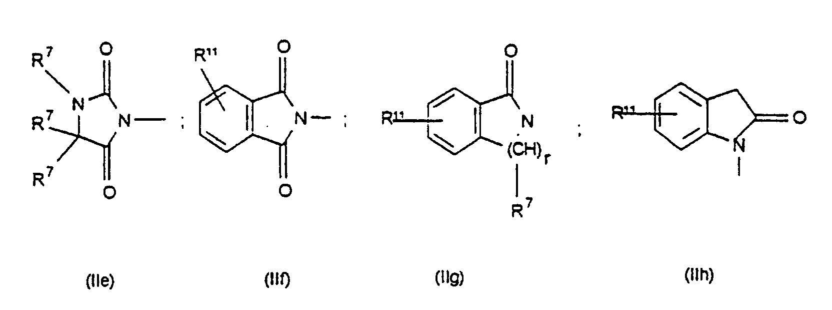 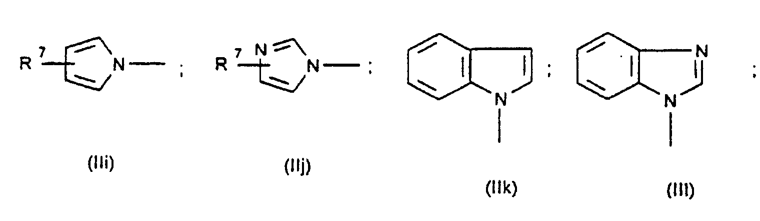 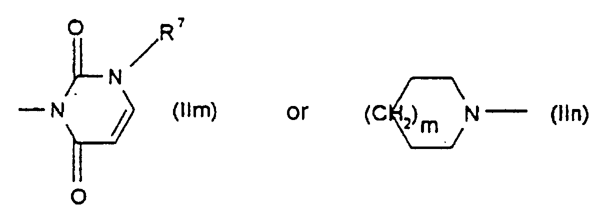 where r is the integer 1 or 2, R¹¹ is a radical as described under 2.1. to 2.15., and R⁷ and m have the abovementioned meaning,
14. -OH,
15. =O or
16. (C₁-C₆)-alkyl-, or
a -C(R)(R³)- radical is -NH- or -NR²-, in which R² is as defined above, and
t is an integer 1, 2, 3 or 4, or
R² and R³ together form a ring with an exocyclic phosphinic or phosphonic acid radical of the subformula II 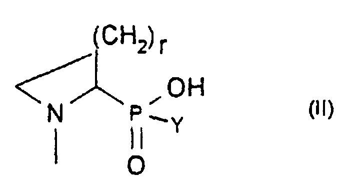 in which r is the integer zero, 1, 2 or 3 and/or one of the carbon atoms in the ring is replaced by -O-, -S- or -(R⁷)N-, in which
R⁷ is
1. a hydrogen atom,
2. (C₁-C₆)-alkyl,
3. phenyl, in which phenyl is unsubstituted or substituted as described under 2.1. to 2.15.,
4. benzyl, in which benzyl is unsubstituted or substituted as described under 2.1. to 2.15., or
5. R²N-C(=NH)-, where R² has the abovementioned meaning,
and/or the carbon atoms in the ring of the subformula II are mono- or polysubstituted by (C₁-C₆)-alkyl-, phenyl-, phenyl-(CH₂)ₘ- or HO-, U is -SO₂- or -CO-,
Y¹ and Y² are identical or different and independently of one another are
a) a hydrogen atom,
b) -OH,
c) -(C₁-C₄)-alkyl, in which alkyl is linear or branched,
d) -(CH₂)ᵤ-phenyl, in which u is zero or 1,
e) -O-(C₁-C₄)-alkyl, in which alkyl is linear or branched, or
f) -O-(CH₂)ₛ-phenyl, in which s is zero or 1,
A is
a) a covalent bond,
b) -O-,
c) -CH=CH- or
d) -C≡C-,
B is
a) -(CH₂)ₒ-, in which o is the integer zero, 1, 2, 3 or 4,
b) -O-(CH₂)ₚ, in which p is an integer from 1 to 5, or
c) -CH=CH-, and
X is -CH=CH-, an oxygen atom or a sulfur atom.

2. A compound of the formula I as claimed in claim 1, wherein
R¹ is
1. phenyl or
2. phenyl which is monosubstituted by
2.1. (C₁-C₆)-alkyl-, in which alkyl is linear, cyclic or branched,
2.2. -OH,
2.3. -C(O)-OH,
2.4. -O-(C₁-C₆)-alkyl,
2.5. pyrrolidine,
2.6. halogen or
2.7. -CF₃, or
3. -O-(C₁-C₆)-alkyl,
R², R⁴ and R⁵ are identical or different and are a hydrogen atom or (C₁-C₆)-alkyl-,
R is a hydrogen atom,
R³ is
1. (C₁-C₆)-alkyl-, in which alkyl is linear, branched or cyclic, and/or in which a hydrogen atom of the alkyl radical is replaced by -OH,
2. R² -S(O)ₙ-(C₁-C₆)-alkyl-, in which R² is (C₁-C₆)-alkyl- or phenyl-(CH₂)ₙ- and n is the integer zero or 1,
3. -(CH₂)ₘ-phenyl, in which phenyl is unsubstituted or mono- or disubstituted as described under 2.1. to 2.15. and/or a hydrogen atom of the -(CH₂)ₘ- chain is replaced by -OH and m is the integer 1, 2, 3, 4 or 5,
4. -(CH₂)ₘ-heteroaryl, in which heteroaryl is as defined under 3.3., 3.5., 3.6., 3.9. or 3.11. and/or is substituted as described under 2.1. to 2.15. and/or a hydrogen atom of the -(CH₂)ₘ- chain is replaced by -OH and m is the integer 1, 2, 3 or 4,
5. a characteristic residue of an amino acid or
6. -(CH₂)ₚ-N(R⁹)(R¹⁰), in which p is an integer zero, 1 or 2, in which R⁹ and R¹⁰ are identical or different and are a hydrogen atom or -SO₂-(CH₂)_{q}-phenyl-phenyl, in which phenyl is unsubstituted or mono- or disubstituted as described under 2.1. to 2.15. and q is the integer zero, 1, 2 or 3, or
7. R⁶-C(O)-, in which R⁶ is
7.1. -OH,
7.2. R²O-, in which R² is as defined above, or
7.3. R⁴-(R⁵)N-, in which R⁴ and R⁵ are as defined above,
8. a hydrogen atom,
9. -OH,
10. =O or
11. (C₁-C₆)-alkyl-, or
a -C(R)(R³)- radical is -NH- or -NR²-, in which R² is as defined above, and
t is an integer 1, 2, 3 or 4,
U is -SO₂-,
Y¹ is -OH,
Y² is
a) -O-(C₁-C₄)-alkyl, in which alkyl is linear or branched,
b) -OH or
c) -(C₁-C₄)-alkyl, in which alkyl is linear or branched,
A is a covalent bond or -O-,
B is a covalent bond or -(C₁-C₄)-alkyl and
X is -CH=CH-.

3. A compound of the formula I as claimed in claim 1 or 2, wherein
R¹ is 1. phenyl which is monosubstituted by halogen,
R² is a hydrogen atom,
R is a hydrogen atom,
R³ is
1. (C₁-C₄)-alkyl-,
2. -phenyl, in which phenyl is unsubstituted or mono- or disubstituted by -CF₃ or -COOH,
3. a hydrogen atom,
4. -OH or
5. -NH-SO₂-phenyl-phenyl, in which phenyl is unsubstituted or substituted by halogen,
t is an integer 1, 2, 3 or 4,
U is -SO₂-,
Y¹ and Y² are -OH or -O-CH₃,
A is a covalent bond,
B is a covalent bond or -(CH₂)ₒ-, in which o is 1, 2 or 3 and
X is -CH=CH-.

4. The compound (R)-[1-(4'-chlorobiphenyl-4-sulfonylamino)-2-methyl-propyl]phosphonic acid, dimethyl [3-(4'-chlorobiphenyl-4-sulfonylamino)-1-hydroxy-3(4-trifluoromethylphenyl)propyl]phosphonate, [1-(4'-chlorobiphenyl-4-sulfonylamino)-3-methylbutyl]phosphonic acid or monoethyl (R,S)-[1-(4'-chlorobiphenyl-4-sulfonylamino)-1-phenyl-methyl]phosphonate.

5. A compound of the formula VI 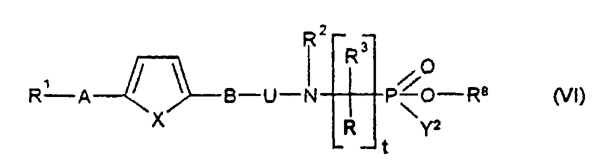 and/or a stereoisomeric form of the compound of the formula VI and/or a physiologically acceptable salt of the compound of the formula VI, where R¹, A, X, B, U, Y², t, R² and R³ have the meaning mentioned in the compound of the formula I as claimed in claim 1 and R⁸ has the meaning mentioned in the compound of the formula IV as claimed in claim 6.

6. A process for preparing the compound of the formula I as claimed in one or more of claims 1 to 5, which comprises
a) reacting an aminophosphinic or -phosphonic acid of the formula III 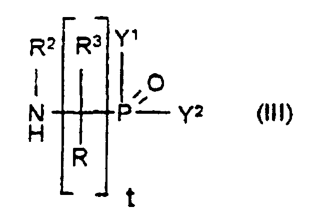 in which R², Y¹, Y², R and R³ are as defined in formula I, with a sulfonic acid or carbonyl derivative of the formula IV 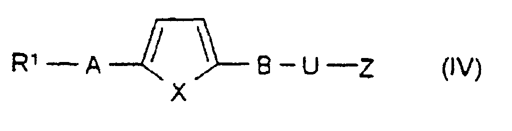 in which R¹, A, X, U and B are as defined in formula I and Z is a halogen atom, imidazolyl or -OR⁸, in which R⁸ is a hydrogen atom, (C₁-C₆)-alkyl, phenyl or benzyl, if appropriate substituted,
in the presence of a base or optionally of a dehydrating agent to give a compound of the formula I, or
b) reacting an aminophosphinic or -phosphonic acid ester of the formula V 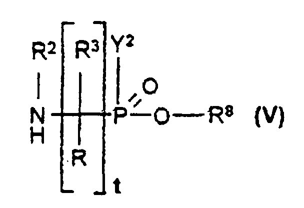 in which R², R³, t, Y² and R⁸ have the abovementioned meaning, with a sulfonic acid or carbonyl derivative of the formula IV to give a compound of the formula VI 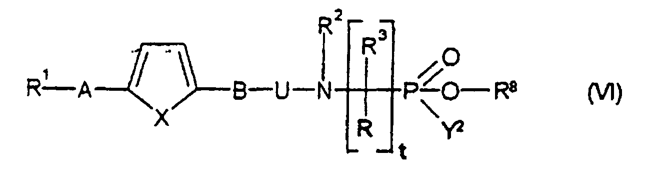 and converting the compound of the formula VI with removal of the radical R⁸, preferably in the presence of a base or acid, into a compound of the formula I, or
c) reacting the compound of the formula VII 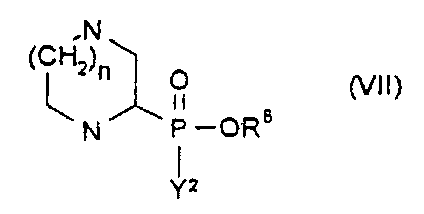 where n is the integer zero, 1 or 2,
with the aid of a protective group E to give a compound of the formula VIII 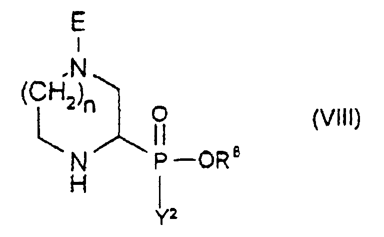 and converting the compound of the formula VIII, using a compound of the formula IV, into a compound of the formula IX 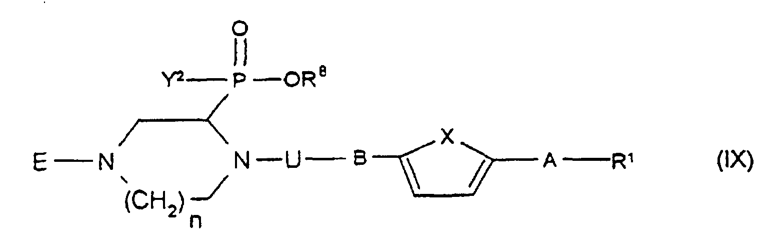 and then converting the compound of the formula IX, with removal of the protective group E and of the radical R⁸ with the aid of suitable cleavage reagents, into the compound of the formula I, or
d) separating a compound of the formula I prepared by one of the processes a), b) or c), which on account of its chemical structure occurs in enantiomeric forms, into the pure enantiomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups, or
e) isolating the compound of the formula I prepared by one of the processes a), b), c) or d) either in free form, or in the case of the presence of acidic or basic groups, converting it into physiologically acceptable salts.

7. A pharmaceutical, comprising an efficacious amount of at least one compound of the formula I as claimed in one or more of claims 1 to 5 together with a pharmaceutically suitable and physiologically acceptable excipient, additive and/or other active compounds and auxiliaries.

8. The use of at least one compound of the formula I as claimed in one or more of claims 1 to 5 for producing pharmaceuticals for the prophylaxis and therapy of disorders in the course of which an increased activity of matrix-degrading metalloproteinases is involved.

9. The use as claimed in claim 8 for the treatment of degenerative joint disorders such as osteoarthroses, spondyloses, chondrolysis after joint trauma or relatively long joint immobilization after meniscus or patella injuries or torn ligaments, disorders of the connective tissue such as collagenoses, periodontal disorders, wound healing disorders and chronic disorders of the locomotory apparatus such as inflammatory, immunological or metabolically related acute and chronic arthritis, arthropathies, myalgias and disorders of the bone metabolism, ulceration, atherosclerosis and stenoses, but also for the treatment of inflammations, carcinomatous disorders, tumor metastasis formation, cachexia, anorexia and septic shock.

10. A process for preparing a pharmaceutical, which comprises bringing at least one compound of the formula I as claimed in one or more of claims 1 to 5 into a suitable administration form using a pharmaceutically suitable and physiologically acceptable excipient and, if appropriate, further suitable active compounds, additives or auxiliaries.

## Revendications

1. Composé de formule I 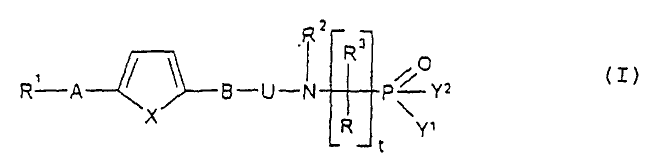 et/ou une forme stéréoisomère du composé de formule I et/ou un sel toléré du point de vue physiologique du composé de formule I, où
R¹ représente des groupes
1. phényle
2. phényle qui est substitué une ou deux fois par des substituants
2.1. alkyle en C₁-C₆, linéaire, cyclique ou ramifié,
2.2. hydroxy,
2.3. (alkyle en C₁-C₆)-C(O)-O-,
2.4. (alkyle en C₁-C₆)-O-,
2.5. (alkyle en C₁-C₆)-(O)-(alkyle en C₁-C₄)-O-,
2.6. halogène,
2.7. -CF₃,
2.8. -CN,
2.9. -NO₂,
2.10. HO-C(O)-,
2.11. (alkyle en C₁-C₆)-O-C(O)-,
2.12. méthylènedioxo,
2.13. R⁴-(R⁵)N-C(O)-,
2.14. R⁴-(R⁵)N- ou
2.15. des composés hétéroaromatiques pris dans les groupes 3.1. à 3.16,
3. un composé hétéroaromatique des groupes 3.1. à 3.16. suivants, qui est non substitué ou substitué comme décrit au points 2.1. à 2.15.,
3.1. pyrrole,
3.2. pyrazole,
3.3. imidazole,
3.4. triazole,
3.5. thiophène,
3.6. thiazole,
3.7. oxazole,
3.8. isoxazole,
3.9. pyridine,
3.10. pyrimidine,
3.11. pyrrolidine,
3.12. indole,
3.13. benzothiophène,
3.14. benzimidazole,
3.15. benzoxazole, ou
3.16. benzothiazole, ou
4. -O-alkyle en C₁-C₆,
R², R⁴ et R⁵ sont identiques ou différents et représentent des groupes
1. atome d'hydrogène,
2. -alkyle en C₁-C₆,
3. HO-C(O)-alkyle en C₁-C₆-,
4. phényl-(CH₂)ₙ-, où phényle est non substitué ou substitué une ou deux fois comme décrit aux points 2.1. à 2.15., ou substitué par un groupe -NH-C(O)-alkyle en C₁-C₃ et n est un entier qui vaut 0, 1 ou 2, ou
5. picolyle ou
6. R⁴ et R⁵ forment, conjointement avec le groupe amino cyclique, un cycle de 4 à 7 chaînons, où éventuellement un des atomes de carbone peut être remplacé par par des groupes -O-, -S- ou -NH- ou deux atomes de carbone adjacents du cycle de 4 à 7 chaînons font partie d'un reste benzyle,
R et R³ sont identiques ou différents et représentent des groupes
1. un atome d'hydrogène,
2. -alkyle en C₁-C₁₀, où alkyle est non substitué, ou un atome d'hydrogène du reste alkyle est remplacé par un groupe -OH,
3. -alcényle en C₂-C₁₀, où alcényle est linéaire ou ramifié,
4. R²-O-alkyle en C₁-C₆-,
5. R²-S-(O)ₙ-alkyle en C₁-C₆- où n possède la signification donnée ci-dessus,
6. R²-S(O)(=NH)-alkyle en C₁-C₆-,
7. un reste de formule IIo 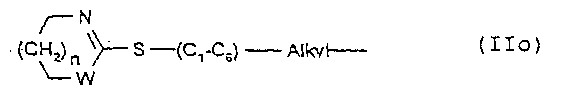 où n est un entier qui vaut 0, 1 ou 2 et W représente un atome d'azote, d'oxygène ou de soufre,
8. phényl-(CH₂)ₘ-, où m est un entier qui vaut 0, 1, 2, 3, 4, 5 ou 6 et/ou un atome d'hydrogène de la chaîne -(CH₂)ₘ- est remplacé par un groupe -OH et phényle est non substitué ou substitué une ou deux fois par
8.1. comme décrit aux points 2.1. à 2.15. et
8.2. -O-(CH₂)ₘ-phényle, où phényle est non substitué ou substitué une ou deux fois comme décrit au point 2.1. à 2.15. et
m est un entier qui vaut 0, 1, 2, 3, 4, 5 ou 6,
8.3. -C(O)-(CH₂)ₘ-phényle, où phényle est défini comme au point 8.2.,
9. hétéroaryl-(CH₂)ₘ-, où hétéroaryle est défini comme aux points 3.1. à 3.16., m est défini comme ci-dessus et/ou un atome d'hydrogène de la chaîne -(CH₂)ₘ- est remplacé par un groupe -OH et hétéroaryle est non substitué ou substitué une ou deux fois par
9.1. comme décrit aux points 2.1. à 2.15.,
9.2. -CH(O),
9.3. -SO₂-phényle, où phényle est non substitué ou défini comme au point 8.2.
9.4. -O-(CH₂)ₘ-phényle,
10. -(CH₂)ₘ-P(O)(OH)-alkyle en C₁-C₃, où m est défini comme ci-dessus,
11. reste caractéristique d'un acide aminé ou
12. R⁶-C(O)-alkyle en C₀-C₆, où R⁶ représente des groupes
1. atome d'hydrogène,
2. -alkyle en C₁-C₆-, où alkyle est linéaire, ramifié ou cyclique,
3. phényle, où phényle est non substitué ou substitué comme décrit aux points 2.1. à 2.15.,
4. hétéroaryle, où hétéroaryle est défini comme aux points 3.1. à 3.16. et/ou substitué comme décrit aux points 2.1. à 2.15. ou substitué par un substituant -(alkyle en C₁-C₄)-COOH,
5. -OH,
6. -OR², où R² possède la signification donnée ci-dessus,
7. -NR⁴-(R⁵), où R⁴ et R⁵ sont définis comme ci-dessus,
8. hétéroaryl-(CH₂)ₘ-NH-, où hétéroaryle est défini comme aux points 3.1. à 3.16. et/ou comme décrit aux points 2.1. à 2.15. et m est défini ci-dessus,
9. R⁴-(R⁵)N-NH-, où R⁴ et R⁵ sont définis comme ci-dessus,
10. HO-C(O)-CH(R³)-NH-, où R³ est défini comme ci-dessus,
13. -(CH₂)ₚ-N(R⁹)(R¹⁰), où p est un entier qui vaut 0, 1, 2, 3 ou 4, où R⁹ et R¹⁰ sont identiques ou différents et représentent des groupes
1. atome d'hydrogène,
2. phényl-(CH₂)ₘ-, où phényle est non substitué ou substitué une ou deux fois comme décrit aux points 2.1. à 2.15 et m est un entier qui vaut 0, 1, 2 ou 3,
3. R^{x}-C(O)-, où R^{x} représente
3.1. -alkyle en C₁-C₆,
3.2. -alcényle en C₂-C₆,
3.3. phényl-CH₂)ₘ-, où phényle est non substitué ou substitué une ou deux fois comme décrit aux points 2.1. à 2.15, et m est un entier qui vaut 0, 1, 2 ou 3, ou
3.4. hétéroaryl-(CH₂)ₘ-, où hétéro-aryle est défini comme aux points 3.1. à 3.16. et/ou substitué comme aux points 2.1. à 2.15. et m est un entier qui vaut 0, 1, 2 ou 3,
4. R^{x}-O-C(O)-, où R^{x} est défini comme ci-dessus,
5. R^{x}-CH(NH₂)-C(O)-, où R^{x} est défini comme ci-dessus,
6. R⁸-N(R⁷)-C(O)-, où R⁸ représente des groupes
6.1. un atome d'hydrogène,
6.2. -alkyle en C₁-C₆,
6.3. phényl-(CH₂)ₘ, où phényle est non substitué ou substitué une ou deux fois comme décrit aux points 2.1. à 2.15, et m est un entier qui vaut 0, 1, 2 ou 3, ou
6.4. hétéroaryl-(CH₂)ₘ-, où hétéro-aryle est défini comme aux points 3.1. à 3.16. et/ou substitué comme aux points 2.1. à 2.15. et m est un entier qui vaut 0, 1, 2 ou 3, et où R⁷ représente un atome d'hydrogène ou un groupe -alkyle en C₁-C₆ ou dans lequel R⁷ et R⁸ forment, conjointement avec l'atome d'azote auquel ils sont liés, un cycle de 4 à 7 chaînons et le cycle est non substitué ou un atome de carbone nucléaire est remplacé par un groupe -O-, -S- ou -NH-,
7. R^{x}-SO₂-, où R^{x} est défini comme ci-dessus,
8. R^{x}-NH-C(=NR⁷)-, où R^{x} et R⁷ sont définis comme ci-dessus, ou représente des groupes
8.1. (alkyle en C₁-C₆)-C(O)-,
8.2. -NO₂ ou
8.3. -SO₂-(CH₂)_{q}-phényle, où phényle est non substitué ou substitué une ou deux fois comme décrit aux points 2.1. à 2.15. et q est un entier qui vaut 0, 1, 2 ou 3,
9. -SO₂-(CH₂)_{q}-phényl-phényle, où phényle est non substitué ou substitué une ou deux fois comme décrit au points 2.1. à 2.15. et q est un entier qui vaut 0, 1, 2 ou 3, ou
10. le reste de formule IIp, 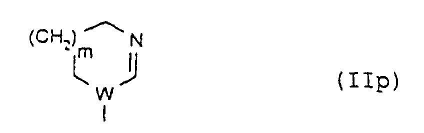 où m est un entier qui vaut 0, 1, 2 ou 3 et W représente un atome d'azote, ou
R⁹ et R¹⁰ forment, conjointement avec l'atome d'azote auquel ils sont liés, un cycle de formules partielles IIa à IIn 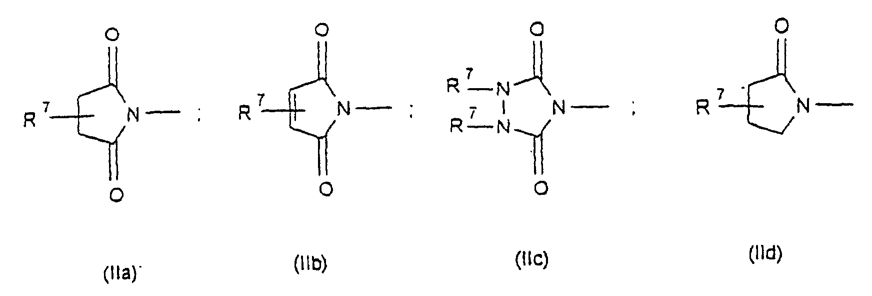 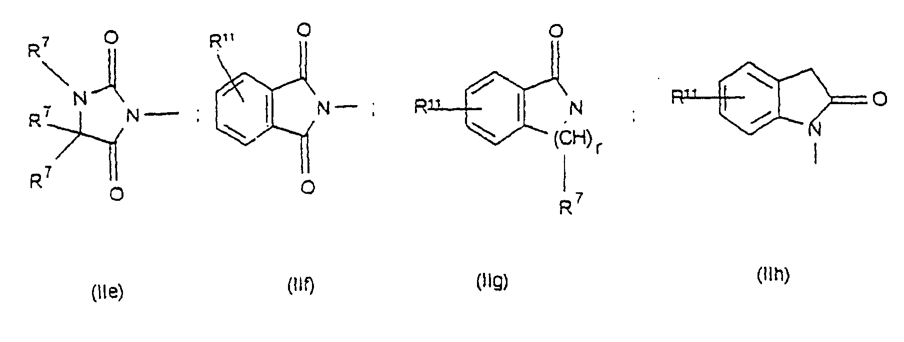 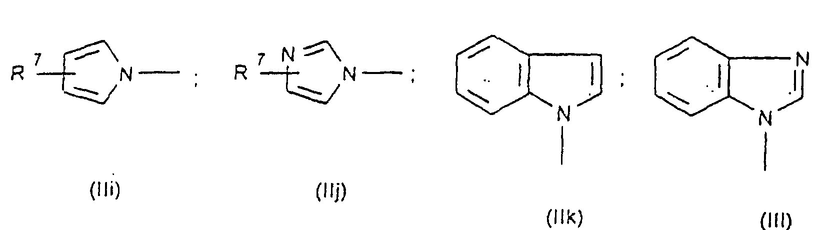 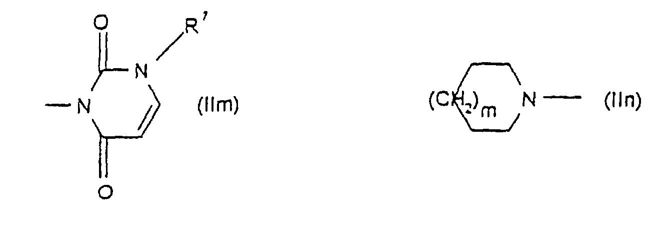 où r est un entier qui vaut 1 ou 2, R¹¹ représente un reste comme décrit aux points 2.1. à 2.15. et R⁷ et m possèdent la signification donnée ci-dessus,
14. -OH,
15. =O ou
16. -alkyle en C₁-C₆, ou un reste de formule -C(R)(R³)-, -NH ou NR²-, où R² est défini comme ci-dessus,
et
t est un entier qui vaut 1, 2, 3 ou 4, ou
R² et R³ forment ensemble un cycle avec un reste exocyclique acide phosphinique ou phosphonique de formule partielle II 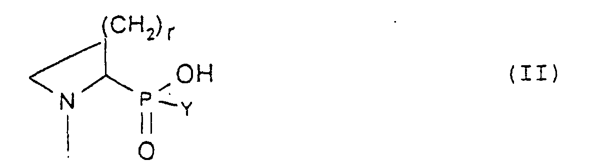 où r est un entier qui vaut 0, 1, 2 ou 3 et/ou un des atomes de carbone dans le cycle est remplacé par des groupes -O-, -S- ou -(R⁷)N-, où
R⁷ représente des groupes
1. atome d'hydrogène,
2. alkyle en C₁-C₆,
3. phényle, où phényle est non substitué ou substitué comme décrit aux points 2.1. à 2.15.,
4. benzyle, où benzyle est non substitué ou substitué comme décrit aux points 2.1. à 2.15., ou
5. R²N-C(=NH)-, où R² possède la signification donnée ci-dessus, et/ou les atomes de carbone dans le cycle de la formule partielle II sont substitués une ou plusieurs fois par des substituants alkyle en C₁-C₆-, phényl-, phényl-(CH₂)ₘ- ou HO-,
U représente des groupes -SO₂- ou -CO-,
Y¹ et Y² sont identiques ou différents et représentent, indépendamment l'un de l'autre, des groupes
a) atome d'hydrogène,
b) -OH,
c) -alkyle en C₁-C₄, où alkyle est linéaire ou ramifié,
d) -(CH₂)ᵤ-phényle, où u vaut 0 ou 1,
e) -O-alkyle en C₁-C₄, où alkyle est linéaire ou ramifié, ou
f) -O-(CH₂)ₛ-phényle, où s vaut 0 ou 1,
A représente des groupes
a) une liaison covalente,
b) -O-,
c) -CH=CH- ou
d) -C≡C-,
B représente des groupes
a) -(CH₂)ₒ-, où o est un entier qui vaut 0, 1, 2, 3 ou 4,
b) -O-(CH₂)ₚ, où p est un entier de 1 à 5, ou
c) -CH=CH-, et
X représente un groupe -CH=CH-, un atome d'oxygène ou un atome de soufre.

2. Composé de formule I selon la revendication 1, **caractérisé en ce que**
R¹ représente des groupes
1. phényle ou
2. phényle qui est substitué une fois par des substituants
2.1. -alkyle en C₁-C₆, où alkyle est linéaire, cyclique ou ramifié,
2.2. -OH,
2.3. -C(O)-OH,
2.4. -O-alkyle en C₁-C₆,
2.5. pyrrolidine,
2.6. halogène ou
2.7. -CF₃, ou
3. -O-alkyle en C₁-C₆,
R², R⁴ et R⁵ sont identiques ou différents et représentent des atomes d'hydrogène ou des groupes -alkyle en C₁-C₆,
R représente un atome d'hydrogène,
R³ représente des groupes
1. -alkyle en C₁-C₆, où alkyle est linéaire, cyclique ou ramifié, et/ou un atome d'hydrogène du reste alkyle est remplacé par un groupe -OH,
2. R²-S(O)ₙ-alkyle en C₁-C₆-, où R² représente des groupes -alkyle en C₁-C₆ ou phényl-(CH₂)ₙ- et n est un entier qui vaut 0 ou 1,
3. phényl-(CH₂)ₙ-, où phényle est non substitué ou substitué une ou deux fois comme décrit aux points 2.1. à 2.15. et/ou un atome d'hydrogène de la chaîne (CH₂)ₘ- est remplacé par un groupe -OH et m est un entier qui vaut 1, 2, 3, 4 ou 5,
4. -(CH₂)ₘ-hétéroaryle, où hétéroaryle possède la signification donnée aux points 3.3, 3.5, 3.6, 3.9 ou 3.11 et/ou substitué comme décrit aux points 2.1 à 2.15. et/ou un atome d'hydrogène de la chaîne -(CH₂)ₘ- est remplacé par un groupe -OH et m est un entier qui vaut 1, 2, 3 ou 4,
5. un reste caractéristique d'un acide aminé ou
6. -(CH₂)ₚ-N(R⁹)(R¹⁰), où p est un entier qui vaut 0, 1 ou 2, où R⁹ et R¹⁰ sont identiques ou différents et représentent un atome d'hydrogène ou un groupe -SO₂-(CH₂)_{q}-phényl-phényle, où phényle est non- substitué ou substitué une ou deux fois comme décrit au points 2.1. à 2.15. et q est un entier qui vaut 0, 1, 2 ou 3, ou
7. R⁶-C(O)-, où R⁶ représente des groupes
7.1. -OH,
7.2. R²O-, où R² est défini comme ci-dessus, ou
7.3. R⁴-(R⁵)N-, où R⁴ et R⁵ sont définis comme ci-dessus,
8. un atome d'hydrogène,
9. -OH,
10. =O ou
11. -alkyle en C₁-C₆, ou un reste -C(R)(R³), -NH- ou -NR², où R² est défini comme ci-dessus, et
t est un entier qui vaut 1, 2, 3 ou 4,
U représente un groupe -SO₂-,
Y¹ représente un groupe -OH,
Y² représente des groupes
a) -O-alkyle en C₁-C₄, où alkyle est linéaire ou ramifié,
b) -OH ou
c) -alkyle en C₁-C₄, où alkyle est linéaire ou ramifié,
A représente une liaison covalente ou -O-,
B représente une liaison covalente ou un groupe -alkyle en C₁-C₄, et
X représente un groupe -CH=CH-.

3. Composé de formule I selon la revendication 1 ou 2, **caractérisé en ce que**
R¹ représente un groupe 1. phényle, qui est substitué une fois par un substituant halogène,
R² représente un atome d'hydrogène,
R représente un atome d'hydrogène,
R³ représente des groupes
1. -alkyle en C₁-C₄,
2. -phényle, où phényle est non substitué ou substitué une ou deux fois par des substituants -CF₃ ou -COOH,
3. un atome d'hydrogène,
4. -OH ou
5. -NH-SO₂-phényl-phényle, où phényle est non substitué ou substitué par un substituant halogène,
t est un entier qui vaut 1, 2, 3 ou 4,
U représente un groupe -SO₂-,
Y¹ et Y² représentent des groupes -OH ou -O-CH₃,
A est une liaison convalente,
B est une liaison covalente ou -(CH₂)ₒ-, où o vaut 1, 2 ou 3, et
X représente un groupe -CH=CH-.

4. Composé l'acide (R)-[1-(4'-chloro-biphényl-4-sulfonylamino)-2-méthylpropyl]-phosphonique, le [3-(4'-chlorobiphényl-4-sulfonylamino)-1-hydroxy-3-(4-trifluorométhyl-phényl)-propyl)-phosphonate de diméthyle, l'acide [1-(4'-chlorobiphényl-4-sulfonylamino)-2-méthylbutyl]-phosphonique ou (R,S)-[1-(4'-chlorobiphényl-4-sulfonylamino)-1-phénylméthyl]-phosphonate de monoéthyle.

5. Composé de formule VI 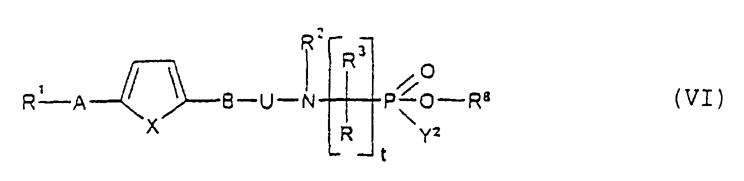 et/ou une forme stéréoisomère du composé de formule VI et/ou un sel toléré du point de vue physiologique du composé de formule VI, où R¹, A, X, B, U, Y², t, R² et R³, qui possèdent la signification donnée à la formule I selon la revendication 1 et R⁸ possède la signification donnée pour le composé de formule VI selon la revendication 6.

6. Procédé pour la préparation du composé de formule I selon une ou plusieurs des revendications 1 à 5, **caractérisé en ce que**
a) un acide aminophosphinique ou -phosphonique de formule III, 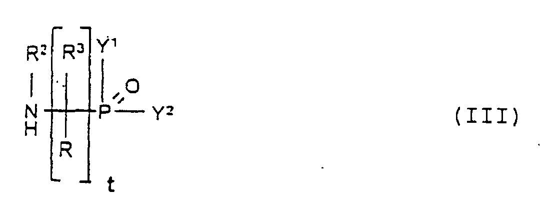 où R², Y¹, Y², R et R³ sont définis comme à la formule I, réagit sur un dérivé de l'acide sulfonique ou de carbonyle de formule IV, 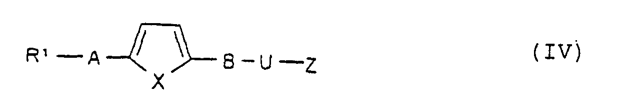 où R¹, A, X, U et B sont définis comme à la formule I et Z représente un atome d'halogène, un groupe imidazolyle ou -OR⁸, où R⁸ représente un atome d'hydrogène, des groupes alkyle en C₁-C₆, phényle ou benzyle, éventuellement substitué,
en présence d'une base ou éventuellement d'un agent déshydratant, pour obtenir un composé de formule I, ou
b) un ester de l'acide aminophosphinique ou -phosphonique de formule V, 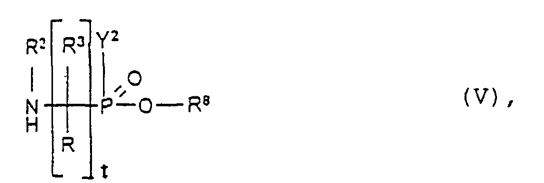 où R², R³, t, Y² et R⁸ possèdent la signification donnée ci-dessus,
réagit sur un dérivé de l'acide sulfonique ou un dérivé de carbonyle de formule IV, pour donner un composé de formule VI 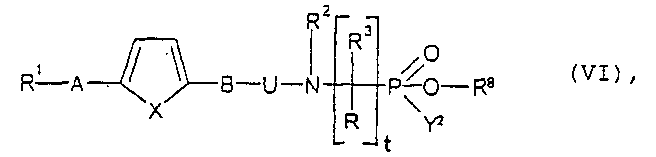 et on transforme le composé de formule VI en éliminant le reste R⁸,
de préférence en présence d'une base ou d'un acide en un composé de formule I, ou
c) on transforme le composé de formule VII, 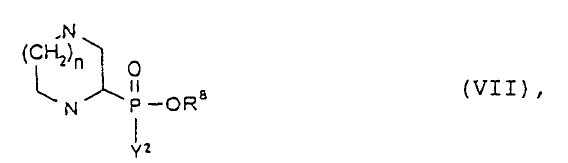 où n est un entier qui vaut 0, 1 ou 2,
à l'aide d'un groupe protecteur E, pour donner un composé de formule VIII, 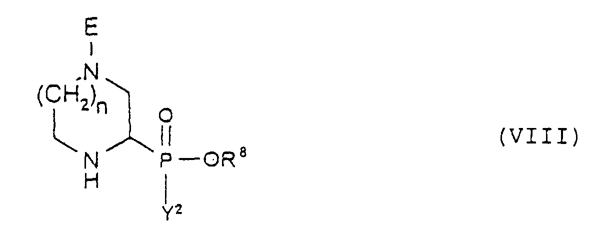 et le composé de formule VIII réagit sur un composé de formule IV pour donner le composé de formule IX, 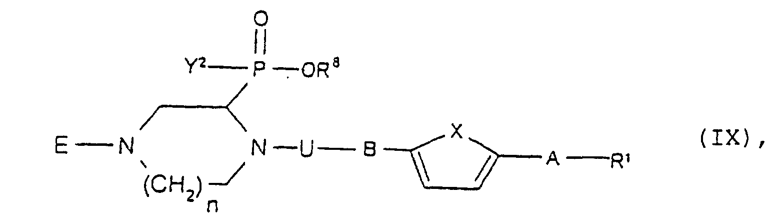 et ensuite on transforme le composé de formule IX en éliminant le groupe protecteur E et le reste R⁸ à l'aide de réactifs de clivage appropriés en transformant en composé de formule I, ou
d) on sépare un composé de formule I préparé selon les procédés a), b), c) ou d) qui se présente, en raison de sa structure chimique sous formes énantiomères, par salification avec des bases ou acides énantiométriquement purs, par chromatographie sur des phases stationnaires chirales ou par transformation à l'aide de composés énantiométriquement purs chiraux comme des acides aminés, la séparation des diastéréoisomères ainsi obtenus, et la séparation des groupes auxiliaires chiraux en les énantiomères purs, ou
e) on isole le composé de formule I préparé selon les procédés a), b), c) ou d) soit sous forme libre, soit on transforme, dans le cas de la présence de groupes acides ou basiques, en sels tolérés du moint de vue physiologique.

7. Médicament, **caractérisé par** une teneur efficace en au moins un composé de formule I selon une ou plusieurs revendications 1 à 5 conjointement avec un véhicule, une matière d'addition et/ou d'autres substances actives et adjuvants pharmaceutiques appropriés et tolérés du point de vue physiologique.

8. Utilisation d'au moins un composé de formule I selon une ou plusieurs des revendications 1 à 5, pour la préparation de médicaments pour la prévention et la thérapie de maladies auxquelles participe une activité accrue de métalloprotéases qui dégradent la matrice.

9. Utilisation selon la revendication 8, pour le traitement de maladies des articulations dégénératives comme des ortéoarthroses, des spondyloses, l'atrophie du cartilage après un traumatisme de l'articulation ou après une immobilisation prolongée de l'articulation suite à des blessures du ménisque ou de la rotule, des périodontites, des troubles de cicatrisation et des maladies chroniques de l'appareil moteur, telles que des arthritides, des arthropathies, des myalgies aiguës et chroniques inflammatoires, immunologiques ou provoquées par des troubles de métabolisme osseux, des ulcérations, l'athérosclérose et des sténoses, ainsi qu'au traitement d'inflammations, de maladies cancéreuses, de formation de métastases tumorales, de cachexie, d'anorexie et de choc septique.

10. Procédé pour la préparation d'un médicament, **caractérisé en ce qu'**on met en une forme d'administration appropriée au moins un composé de formule I selon une ou plusieurs des revendications 1 à 5, avec un véhicule pharmaceutique approprié et toléré du point de vue physiologique ainsi que d'autres substances actives, matières d'addition ou adjuvants.
